# FASCICULE DE BREVET EUROPEEN

(11) **EP 1 951 225 B1**
(45) Date de publication et mention de la délivrance du brevet: **01.02.2012**
(21) Numéro de dépôt: 06841810.2
(22) Date de dépôt: 17.11.2006
(51) Int. Cl.: A61K 31/404, C07D 209/12, C07D 209/14, A61P 43/00

(54) **DERIVES DE 3-ACYLINDOLE, LEUR PREPARATION ET LEUR APPLICATION EN THERAPEUTIQUE**
3-ACYLINDOL-DERIVATE SOWIE DEREN HERSTELLUNG UND THERAPEUTISCHE ANWENDUNG
3-ACYLINDOLE DERIVATIVES THE PREPARATION AND THE THERAPEUTIC USE THEREOF

(30) Priorité: 18.11.2005 FR 0511740
(43) Date de publication de la demande: 06.08.2008
(73) Titulaire: SANOFI, 75013 Paris (FR)
(72) Inventeur: BARTH, Francis, F-92160 Antony (FR); VERNHET, Claude, F-92160 Antony (FR); RINALDI-CARMONA, Murielle, F-92160 Antony (FR); GUILLAUMONT-LEGEAY, Carole, Elisabeth, F-92160 Antony (FR)
(74) Mandataire: Kugel, Dominique
(86) Numéro de dépôt international: PCT/FR2006/002535
(87) Numéro de publication internationale: WO 2007/057571

(56) Documents cités:
- WO-A-2006/069196
- WO-A1-02/42269
- WO-A2-03/097597
- US-A- 5 532 237
- HUFFMAN ET AL: "1-Pentyl-3-phenylacetylindoles, a new class of cannabimimetic indoles" BIOORGANIC & MEDICINAL CHEMISTRY LETTERS, OXFORD, GB, vol. 15, no. 18, 15 septembre 2005 (2005-09-15), pages 4110-4113, XP005021108 ISSN: 0960-894X

## Description

La présente invention a pour objet de nouveaux dérivés de 3-acylindole, ayant une affinité pour les récepteurs CB₂ des cannabinoïdes, à leur préparation et à leur application en thérapeutique.

Le Δ⁹-THC est le principal constituant actif extrait de *Cannabis sativa* [Paton, Annual Review in Pharmacology (1975) 15 : 191-220]

De nombreux articles ont décrit non seulement des effets psychotropes des cannabinoïdes, mais aussi une influence de ces derniers sur la fonction immunitaire [Klein et al., Immunology Today (1998) 19: 373-381], le contrôle de la douleur [Pertwee, Progress in Neurobiology (2001) 63 : 569-611], de la prise alimentaire [Cota et al. International Journal of Obesity (2003) 27 : 289-301] et de nombreuses autres fonctions biologiques [Nahas et al. Marihuana and medicine (1999) Humana Press : Totowa, New Jersey, USA].

Les effets des cannabinoïdes sont dus à une interaction avec des récepteurs spécifiques de haute affinité, couplés aux protéines G, présents au niveau central et périphérique [Howlett et al., Pharmacological Reviews (2002) 54 : 161-2002].

Les effets centraux des cannabinoïdes relèvent d'un premier type de récepteur des cannabinoïdes (CB₁) présent principalement dans le cerveau mais aussi à la périphérie [Matsuda et al., Nature (1990) 346: 561-564]. Par ailleurs, Munro et al. [Nature (1993) 365 : 61-65] ont cloné un second type de récepteur aux cannabinoïdes appelé CB₂ qui est présent à la périphérie et en particulier dans les cellules du système immunitaire.

Certains dérivés indoliques ont été cités dans l'art antérieur comme présentant une affinité pour les récepteurs CB₂.

Ainsi, le brevet US 5 532 237 décrit des composés de formule : 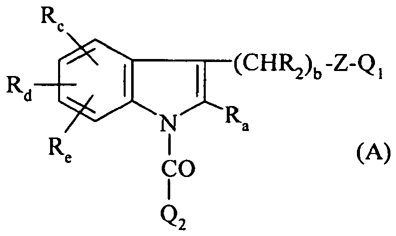 dans laquelle les substituants ont différentes valeurs.
La demande de brevet EP 833 818 décrit des composés de formule : 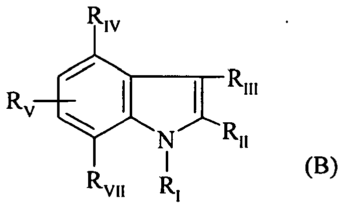 dans laquelle les substituants ont différentes valeurs.

Par ailleurs, le brevet US 4 581 354 décrit des dérivés indoliques actifs comme analgésiques et anti-inflammatoires de formule : 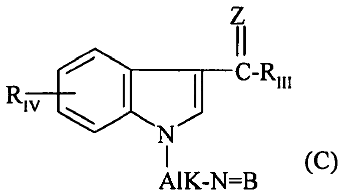 dans laquelle Z peut représenter l'oxygène ou un groupe NOH.

La demande de brevet internationale WO 2002/42269 décrit des dérivés d'aroylindole de formule : 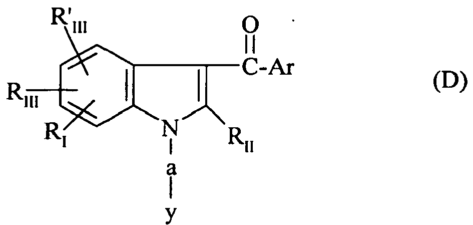 dans laquelle les substituants ont différentes valeurs.

La demande de brevet internationale W02003/097597 décrit des composés de formule : 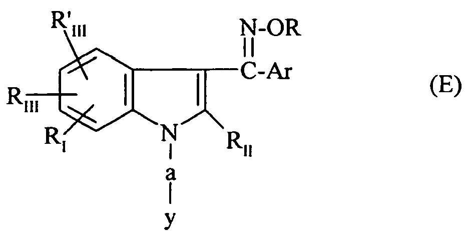 dans laquelle les substituants ont différentes valeurs.

La demande de brevet internationale WO 2006/069 196 décrit des composés de formule : 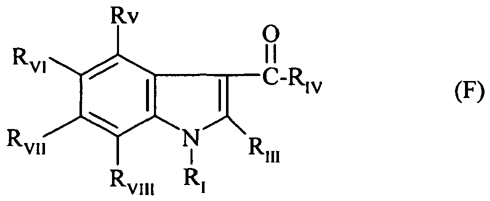 dans laquelle les substituants ont différentes valeurs.

La présente invention a pour objet des composés répondant à la formule (I) : 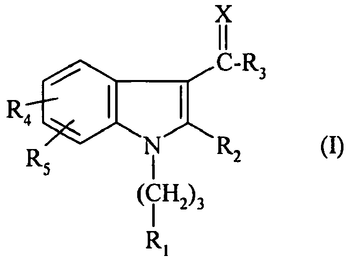 dans laquelle :
- X représente un atome d'oxygène ;
- R₁ représente un groupe choisi parmi -N(R₇)SO₂R₈, -N(R₇)SO₂NR₉R₁₀, -SR₈, - SOR₈, -SO₂R₈, -SO₂NR₉R₁₀ ;
- R₂ représente un atome d'hydrogène ou un (C₁-C₄)alkyle ;
- R₃ représente :
   un (C₁ -C₆)alkyle non substitué ou substitué par :
      a) un ou plusieurs atomes d'halogène ;
      b) un carbocycle non aromatique en (C₃-C₁₂), saturé ou insaturé, non substitué ou substitué une ou plusieurs fois par un (C₁-C₄)alkyle ;
      c) un phényle non substitué ou substitué une ou plusieurs fois par des substituants choisis indépendamment parmi un atome d'halogène, un groupe Alk, un groupe OAlk, un groupe -S(O)ₙAlk, un nitro, un cyano ;
      d) un naphtyle non substitué ou substitué une ou plusieurs fois par des substituants choisis indépendamment parmi un atome d'halogène, un (C₁-C₄)alkyle, un (C₁-C₄)alcoxy, un radical trifluorométhyle ;
   un benzhydrylméthyle ;
- R₄ et R₅ représentent chacun indépendamment un atome d'hydrogène, un atome d'halogène, un (C₁-C₄)alkyle, un (C₁-C₄)alcoxy, un radical trifluorométhyle, un nitro ;
- R₇ représente un atome d'hydrogène ou un (C₁-C₄)alkyle ;
- R₈ représente un (C₁-C₄)alkyle ou un radical trifluorométhyle ;
- R₉ et R₁₀ représentent chacun indépendamment un atome d'hydrogène ou un (C₁-C₄)alkyle ;
- n représente 0, 1 ou 2;
- Alk représente un (C₁-C₄) alkyle non substitué ou substitué une ou plusieurs fois par un atome de fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Lorsque les composés de formule (I) comprennent un atome de soufre, tous les isomères optiques ainsi que leur mélange en proportions quelconques sont objets de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₄)alkyle ou respectivement (C₁-C₆)alkyle, on entend un radical alkyle linéaire ou ramifié de un à quatre atomes de carbone ou respectivement de un à six atomes de carbone, tel que le radical méthyle, éthyle, propyle, isopropyle, butyle, isobutyle, *sec*-butyle, *tert*-butyle, pentyle, isopentyle, hexyle, isohexyle.

Par (C₁-C₄)alcoxy on entend un radical alcoxy linéaire ou ramifié de un à quatre atomes de carbone tel que le radical méthoxy, éthoxy, propoxy, isopropoxy, butoxy, *sec*-butoxy, *tert*-butoxy.

Les radicaux carbocycliques non aromatiques en C₃-C₁₂, saturé ou insaturé, comprennent les radicaux mono ou polycycliques, condensés, pontés ou spiraniques. Les radicaux monocycliques incluent les cycloalkyles par exemple cyclopropyle, cyclobutyle, cyclopentyle, cyclohexyle, cycloheptyle, cyclooctyle ou les cycloalcènes par exemple cyclohexène. Les radicaux di- ou tricycliques condensés, pontés ou spiraniques, incluent par exemple les radicaux norbornyle, bornyle, isobomyle, noradamantyle, adamantyle, spiro[5.5]undécyle, bicyclo[2.2.1] heptyle, bicyclo[3.2.1]octyle ; bicyclo[3.1.1]heptyle.

Parmi les composés de formule (I), objets de l'invention, un premier groupe de composés est constitué par les composés pour lesquels :
- X représente un atome d'oxygène ;
- R₁ représente un groupe choisi parmi -NHSO₂CH₃, -NHSO₂CF₃, -NHSO₂N(CH₃)₂, -S-CH₃, SO-CH₃, -SO₂-CH₃ ;
- R₂ représente un atome d'hydrogène, un méthyle, un éthyle ;
- R₃ représente :
   un *tert*-butyle, un isobutyle, un 2,2-diméthylpropyle, un adamantylméthyle, un phénylméthyle, un (2-bromophényl)méthyle, un (2-chlorophényl)méthyle, un (2-fluorophényl)méthyle, un (3-méthoxyphényl)méthyle, un [2-(trifluorométhyl) phényl]méthyle, un [3-(trifluorométhyl)phényl]méthyle, un [4-(trifluorométhyl) phényl]méthyle, un (2,4-dichlorophényl)méthyle, un (2,3-difluorophényl) méthyle, un [2-fluoro-3-(trifluorométhyl)phényl]méthyle, un [2-fluoro-5-(trifluorométhyl)phényl]méthyle, un [3-fluoro-5-(trifluorométhyl)phényl] méthyle, un 1-méthyl-1-phényléthyle, un 2-phényléthyle, un 2-(2-chlorophényl) éthyle, un 3-phénylpropyle, un naphtylméthyle, un (2-méthylphényl)méthyle, un (2-nitrophényl)méthyle, un [3-(trifluorométhylthio)phényl]méthyle, un (2-méthoxyphényl)méthyle, un [2-(trifluorométhylthio)phényl]méthyle, un [2-(trifluorométhoxy)phényl]méthyle, un (2-cyanophényl)méthyle ;
   un benzhydrylméthyle ;
- R₄ représente un atome d'hydrogène ou est en position -6- de l'indole et représente un atome de chlore ou un méthyle ;
- R₅ représente un atome d'hydrogène ou est en position -7- de l'indole et représente un atome de brome, de chlore ou de fluor, un méthoxy, un nitro ; ainsi que leurs hydrates ou leurs solvats.

Parmi les composés de formule (I) objets de l'invention, on peut notamment citer les composés suivants :
- N-[3-[7-bromo-3-(3,3-diméthylbutanoyl)-2-méthyl-1*H*-indol-1-yl]propyl]méthanesulfonamide ;
- N-[3-[7-bromo-2-méthyl-3-[[2-(trifluorométhyl)phényl]acétyl]-1*H*-indol-1-yl]propyl]méthanesulfonamide ;
- N-[3-[7-chloro-3-(3,3-diméthylbutanoyl)-2-méthyl-1*H*-indol-1-yl]propyl]méthanesulfonamide ;
- N-[3-[3-(1-adamantylacétyl)-7-chloro-2-méthyl-1*H*-indol-1-yl]propyl]méthanesulfonamide ;
- N-[3-[3-[(2-bromophényl)acétyl]-7-chloro-2-méthyl-1*H*-indol-1-yl]propyl]méthanesulfonamide ;
- N-[3-[7-chloro-3-[(2-chlorophényl)acétyl]-2-méthyl-1*H*-indol-1-yl]propyl]méthanesulfonamide ;
- N-[3-[7-chloro-2-méthyl-3-[[2-(trifluorométhyl)phényl]acétyl]-1*H-*indol-1-yl]propyl]méthanesulfonamide ;
- N-[3-[7-chloro-3-[(2,3-difluorophényl)acétyl]-2-méthyl-1*H*-indol-1-yl]propyl]méthanesulfonamide ;
- N-[3-[7-fluoro-2-méthyl-3-[[2-(trifluorométhyl)phényl]acétyl]-1*H*-indol-1-yl]propyl]méthanesulfonamide ;
- N-[3-[7-bromo-3-(3,3-diméthylbutanoyl)-2,6-diméthyl-1*H*-indol-1-yl]propyl]méthanesulfonamide ;
- N-[3-[7-bromo-3-[(2-chlorophényl)acétyl]-2,6-diméthyl-1*H*-indol-1-yl]propyl]méthanesulfonamide ;
- 1-[7-chloro-2-méthyl-1-[3-(méthylthio)propyl]-1*H*-indol-3-yl)-2-[2-(trifluorométhyl)phényl]éthanone ;
- 2-(2-bromophényl)-1-[6,7-dichloro-2-méthyl-1-[3-(méthylthio)propyl]-1*H-*indol-3-yl]éthanone oxime;
- 2-(1-adamantyl)-1-[6,7-dichloro-2-méthyl-1-[3-(méthylthio)propyl]-1*H*-indol-3-yl]éthanone ;
- 2-(1-adamantyl)-1-[6,7-dichloro-2-méthyl-1-[3-(méthylsulfinyl)propyl]-1*H-*indol-3-yl]éthanone ;
   N-[3-[7-bromo-3-[(2-chlorophényl)acétyl]-2-méthyl-1*H*-indol-1-yl]propyl]méthanesulfonamide ;
- N-[3-[7-bromo-3-[(2-fluorophényl)acétyl]-2-méthyl-1*H*-indol-1-yl]propyl]méthanesulfonamide ;
- N-[3-[7-bromo-2-méthyl-3-[(2-méthylphényl)acétyl]-1*H*-indol-1-yl]propyl]méthanesulfonamide ;
- N-[3-[7-bromo-2-méthyl-3-[(2-nitrophényl)acétyl]-1*H*-indol-1-yl]propyl]méthanesulfonamide ;
- N-[3-[7-chloro-2-méthyl-3-[(2-méthylphényl)acétyl]-1*H*-indol-1-yl]propyl]méthanesulfonamide ;
- N-[3-[7-chloro-3-[(2-méthoxyphényl)acétyl]-2-méthyl-1*H*-indol-1-yl]propyl]méthanesulfonamide ;
- N-[3-[7-chloro-2-méthyl-3-[[2-[(trifluorométhyl)thio]phényl]acétyl]-1*H*-indol-1-yl]propyl]méthanesulfonamide ;
- N-[3-[3-[(2-bromophényl)acétyl]-7-fluoro-2-méthyl-1*H*-indol-1-yl]propyl]méthanesulfonamide ;
- N-[3-[3-[(2-chlorophényl)acétyl]-7-fluoro-2-méthyl-1*H*-indol-1-yl]propyl]méthanesulfonamide ;
- N-[3-[7-fluoro-3-[(2-fluorophényl)acétyl]-2-méthyl-1*H*-indol-1-yl]propyl]méthanesulfonamide ;
- N-[3-[7-fluoro-2-méthyl-3-[(2-méthylphényl)acétyl]-1*H*-indol-1-yl]propyl]méthanesulfonamide ;
- N-[3-[7-fluoro-3-[(2-méthoxyphényl)acétyl]-2-méthyl-1*H*-indol-1-yl]propyl]méthanesulfonamide ;
- N-[3-[7-bromo-2,6-diméthyl-3-[[2-(trifluoroméihyl)phényl]acétyl]-1*H*-indol-1-yl]propyl]méthanesulfonamide ;
- N-[3-[3-[(2-bromophényl)acétyl]-7-méthoxy-2-méthyl-1*H*-indol-1-yl]propyl]méthanesulfonamide ;
- N-[3-(7-méthoxy-2-méthyl-3-[[2-(trifluorométhyl)phényl]acétyl]-1*H*-indol-1-yl]propyl]méthanesulfonamide ;
- N-[3-[3[(2-chlorophényl)acétyl]-7-nitro-1*H*-indol-1-yl]propyl]méthanesulfonamide ;
- N-[3-[3[(2-fluorophényl)acétyl]-7-nitro-1*H*-indol-1-yl]propyl]méthanesulfonamide ;
- N-[3-[3[(2-méthylphényl]acétyl]-7-nitro-1*H*-indol-1-yl]propyl]méthanesulfonamide ;
- N-[3-(7-nitro-3-[[2-(trifluorométhyl)phényl)acétyl]-1*H*-indol-1-yl]propyl]méthanesulfonamide ;
- N-[3-[3-[(2-bromophényl)acétyl]-7-nitro-1*H*-indol-1-yl]propyl]méthanesulfonamide ;
- N-[3-[3-[(2-méthoxyphényl)acétyl]-7-nitro-1*H*-indol-1-yl]propyl]méthanesulfonamide ;
- N-[3-[7-nitro-3-[[(2-(trifluorométhoxy)phényl]acétyl]-1*H*-indol-1-yl]propyl]méthanesulfonamide ;
- N-[3-[7-nitro-3-[(2-(nitrophényl)acétyl]-1*H*-indol-1-yl]propyl]méthanesulfonamide ;
- N-[3-[7-bromo-3-[(2-bromophényl)acétyl]-2-méthyl-1*H*-indol-1-yl]propyl]méthanesulfonamide ;
- N-[3-[7-bromo-3-[(2-(méthoxyphényl)acétyl]-2-méthyl-1*H*-indol-1-yl]propyl]méthanesulfonamide ;
- N-[3-[7-bromo-2-méthyl-3-[[2-(trifluorométhoxy)phényl]acétyl]-1*H*-indol-1-yl]propyl]méthanesulfonamide ;
- N-[3-[7-fluoro-2-méthyl-3-[[2-(trifluorométhoxy)phényl]acétyl]-1*H*-indol-1-yl]propyl]méthanesulfonamide ;
- N-[3-[7-fluoro-2-méthyl-3-[(2-nitrophényl)acétyl]-1*H*-indol-1-yl]propyl]méthanesulfonamide ;
- 1,1,1-trifluoro-N-[3-[7-fluoro-2-méthyl-3-(2-(trifluorométhyl)phényl]acétyl]-1*H*-indol-1-yl]propyl]méthanesulfonamide ;
ainsi que leurs hydrates ou leurs solvats.

Conformément à l'invention, on peut préparer les composés de formule (I) dans laquelle X représente un atome d'oxygène selon un procédé qui est caractérisé en ce que :

On fait réagir en présence d'un acide de Lewis, un composé de formule : 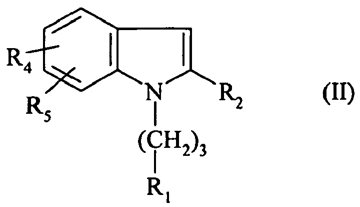 dans laquelle R₁, R₂, R₄ et R₅ sont tels que définis pour un composé de formule (I); avec un halogénure d'acide de formule : 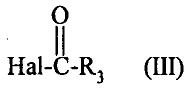 dans laquelle R₃ est tel que défini pour un composé de formule (I) et Hal représente un atome d'halogène.

La réaction s'effectue dans les conditions de Friedel et Crafts en présence d'un acide de Lewis tel que le chlorure d'aluminium ou le dichlorure d'éthylaluminium, dans un solvant tel que le dichlorométhane ou le toluène, à une température comprise entre -30°C et la température ambiante, selon le procédé décrit dans J. Med. Chem., 1995, 38, 3094.

Selon une variante du procédé ci-dessus on peut préparer un composé de formule (I) dans laquelle R₁ = -SOR₈ ou -SO₂R₈ par réaction d'un composé de formule (I) dans laquelle R₁ = -SR₈ avec un agent oxydant. Comme agent oxydant, on peut utiliser l'eau oxygénée ou l'acide 3-chloroperbenzoïque, dans un solvant tel que le dichlorométhane et à une température comprise entre 0°C et la température ambiante.

Selon le nombre d'équivalents d'oxydant utilisé et selon la température de la réaction, on obtient un sulfoxyde (R₁ = -SOR₈) ou une sulfone (R₁ = -SO₂R₈). On peut également obtenir un mélange des deux composés que l'on sépare en utilisant des procédés connus de l'homme du métier, par exemple, par chromatographie préparative.

Les composés de formule (I) ainsi obtenu peuvent être ultérieurement séparés du milieu réactionnel et purifiés selon les méthodes classiques, par exemple, par cristallisation ou chromatographie.

Les composés de formule (II) sont connus et se préparent selon des méthodes connues telles que celles décrites dans WO 02/42269 ou dans WO 03/097597.

Les composés de formule (III) sont connus, commerciaux ou préparés selon des méthodes connues.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
MeOH : méthanol
DCM : dichlorométhane
AcOEt : acétate d'éthyle
DIPEA : diisopropyléthylamine
TFA : acide trifluoroacétique
F : point de fusion
TA : température ambiante
CLHP : chromatographie liquide haute performance

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, qd : quadruplet, m : massif, mt : multiplet, se : singulet élargi, dd : doublet dédoublé.

Les composés selon l'invention sont analysés par couplage LC/UV/MS (chromatographie liquide/détection UV/spectrométrie de masse). On mesure le pic moléculaire (MH⁺) et le temps de rétention (tr) en minutes. Les conditions utilisées sont les suivantes :

On utilise une colonne symmetry C₁₈ de 2,1 x 50 mm, 3,5 µm, à 30°C, débit 0,4 ml / minute.

L'éluant est composé comme suit :
- Solvant A : 0,005% d'acide trifluoroacétique dans l'eau à pH = 3,15 ;
- Solvant B : 0,005% d'acide trifluoroacétique dans l'acétonitrile ;

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection s'effectue à λ = 210 nm et l'enregistrement des spectres de masse est effectué en mode electrospray (ESI) positif, afin d'observer les ions issus de la protonation des composés analysés (MH⁺).

### PREPARATIONS

### 1. Préparations des composés de formule (II).

### Préparation 1.1

N-[3-(2-Méthyl-1*H*-indol-1-yl)propyl]méthanesulfonamide.
(II) : R₁ = -NHSO₂CH₃ ; R₂ = -CH₃ ; R₄ = H ; R₅ = H.

On prépare ce composé décrit dans le TABLEAU 3 à la Préparation 3.22 dans WO 02/42269 selon les méthodes citées.

### Préparation 1.2

N-[3-(7-Bromo-2-méthyl-1*H*-indol-1-yl)propyl]méthanesulfonamide.
(II) : R₁ = -NHSO₂CH₃ ; R₂ = -CH₃ ; R₄ = H ; R₅ = 7-Br.

On prépare ce composé décrit dans le TABLEAU 3 à la Préparation 3.5 dans WO 02/42269 selon les méthodes citées.

### Préparation 1.3

N-[3-(7-Chloro-2-méthyl-1*H*-indol-1-yl)propyl]méthanesulfonamide.
(II) : R₁ = -NHSO₂CH₃ ; R₂ = -CH₃ ; R₄ = H ; R₅ = 7-Cl.

On prépare ce composé selon le mode opératoire décrit à l'étape B de l'Exemple 3 dans WO 02/42269.

### Préparation 1.4

N-[3-(7-Chloro-2-éthyl-1*H*-indol-1-yl)propyl]méthanesulfonamide.
(II) : R₁ = -NHSO₂CH₃ ; R₂ = -CH₂CH₃ ; R₄ = H ; R₅ = 7-Cl.

On prépare ce composé décrit dans le TABLEAU 3 à la Préparation 3.9 dans WO 02/42269 selon les méthodes citées.

### Préparation 1.5

N-[3-(7-Fluoro-2-méthyl-1*H*-indol-1-yl)propyl]méthanesulfonamide.
(II) : R₁ = -NHSO₂CH₃ ; R₂ = -CH₃ ; R₄ = H ; R₅ = 7-F.

### A) -Fluoro-2-méthyl-1H-indole.

On refroidit à -25°C, sous atmosphère d'azote, 1600 ml d'une solution 0,5 M de bromure d'isopropènylmagnésium dans le THF puis ajoute, goutte à goutte, une solution de 37,65 g de 2-fluoronitrobenzène dans 250 ml de THF et laisse 1 heure sous agitation à -20°C. On verse le mélange réactionnel dans une solution saturée de NH₄Cl, extrait la phase aqueuse à l'éther, et concentre les phases organiques jointes sous vide. On reprend le résidu dans l'eau, extrait au DCM, lave la phase organique à l'eau, par une solution saturée de NaCl, sèche sur MgSO₄ et évapore le solvant sous vide. On chromatographie le résidu sur gel de silice en éluant par le mélange cyclohexane/AcOEt (95/5 ; v/v). On obtient 12,35 g du composé attendu.

### B) -(3-Chloropropyl)-7-fluoro-2-méthyl-1H-indole.

A un mélange de 15,64 g de KOH pilée dans 300 ml de toluène on ajoute une solution de 10,4 g du composé obtenu à l'étape précédente dans 170 ml de toluène puis 1,04 g de tétrabutylammonium hydrogénosulfate et chauffe à reflux pendant 30 minutes. On ajoute ensuite 27,6 ml de 1-bromo-3-chloropropane et poursuit le reflux pendant 3 heures. On verse le mélange réactionnel dans l'eau, lave la phase organique à l'eau, par une solution saturée de NaCl, sèche sur Na₂SO₄ et évapore le solvant sous vide. On obtient 16,5 g du composé attendu.

### C) -Fluoro-1-(3-iodopropyl)-2-méthyl-1H-indole.

A une solution de 16,5 g du composé obtenu à l'étape précédente dans 900 ml d'acétonitrile on ajoute 76,9 g de NaI et chauffe à reflux pendant 3 jours. On concentre sous vide, reprend le résidu à l'eau, extrait au toluène, lave la phase organique à l'eau, par une solution saturée de NaCl, sèche sur MgSO₄ et évapore le solvant sous vide. On obtient 20,8 g du composé attendu.

### D) -[3-(7-Fluoro-2-méthyl-1H-indol-1-yl)propyl]méthanesulfonamide.

Sous atmosphère d'azote, on introduit 3,53 g d'hydrure de sodium à 60 % dans l'huile dans 250 ml de DMF, refroidit à 5°C puis ajoute une solution de 8,4 g de méthanesulfonamide dans 125 ml de DMF et laisse 10 minutes sous agitation à 5°C. On ajoute ensuite une solution de 7 g du composé obtenu à l'étape précédente dans 125 ml de DMF et laisse 4 heures sous agitation en laissant remonter la température à TA. On verse le mélange réactionnel dans l'eau, extrait au DCM, lave la phase organique à l'eau, par une solution saturée de NaCl, sèche sur MgSO₄ et évapore le solvant sous vide. On chromatographie le résidu sur gel de silice en éluant par le mélange cyclohexane/AcOEt (60/40 ; v/v). On obtient 4,4 g du composé attendu.

### Préparation 1.6

N-[3-(7-Bromo-2,6-diméthyl-1*H*-indol-1-yl)propyl]méthanesulfonamide.
(II) : R₁ = -NHSO₂CH₃ ; R₂ = -CH3 ; R₄ = 6-CH₃ ; R₅ = 7-Br.

On prépare ce composé décrit dans le TABLEAU 3 à la Préparation 3.24 dans WO 02/42269 selon les méthodes citées.

### Préparation 1.7

N-[3-(6,7-Dichloro-1*H*-indol-1-yl)propyl]méthanesulfonamide.
(II) : R₁ = -NHSO₂CH₃ ; R₂ = H ; R₄ = 6-Cl ; R₅ = 7-Cl.

### A) 6,7-Dichloro-1H-indole.

On refroidit à -45°C, sous atmosphère d'azote, 800 ml d'une solution 1M de bromure de vinylmagnésium dans le THF puis ajoute, goutte à goutte, une solution de 51,2 g de 2,3-dichloronitrobenzène dans 1000 ml de THF et laisse 1 heure sous agitation à -30°C. On verse le mélange réactionnel sur 1000 ml d'une solution saturée de NH₄Cl, extrait à l'éther, lave les phases organiques jointes à l'eau, par une solution saturée de NaCl, sèche sur MgSO₄ et évapore les solvants sous vide. On chromatographie le résidu sur gel de silice en éluant par le mélange cyclohexane/AcOEt (95/5 ; v/v). On obtient 27,75 g du composé attendu.

### B) 6,7-Dichloro-1-(3-chloropropyl)-1H-indole.

A un mélange de 33,5 g de KOH pilée dans 600 ml de toluène on ajoute une solution de 27,75 g du composé obtenu à l'étape précédente dans 600 ml de toluène puis 2,8 g de tétrabutylammonium hydrogénosulfate et chauffe à reflux pendant 30 minutes. On ajoute ensuite 50 ml de 1-bromo-3-chloropropane et poursuit le reflux pendant 2 heures. Après refroidissement à TA, on verse le mélange réactionnel dans l'eau, lave la phase organique à l'eau, par une solution saturée de NaCl, sèche sur MgSO₄ et évapore le solvant sous vide. On obtient 39,2 g du composé attendu.

### C) 6,7-Dichloro-1-(3-iodopropyl)-1H-indole.

A une solution de 39,2 g du composé obtenu à l'étape précédente dans 1200 ml d'acétonitrile on ajoute 156,6 g de NaI et chauffe à reflux pendant 3 jours. On concentre sous vide, reprend le résidu à l'eau, extrait au toluène, lave la phase organique à l'eau, par une solution saturée de NaCl, sèche sur MgSO₄ et évapore le solvant sous vide. On obtient 46,85 g du composé attendu.

### D)N-[3-(6,7-Dichloro-1H-indol-1-yl)propyl]méthanesulfonamide.

On refroidit à 5°C un mélange de 4,5 g d'hydrure de sodium à 60 % dans l'huile dans 250 ml de DMF, ajoute, goutte à goutte, une solution de 10,7 g de méthanesulfonamide dans 120 ml de DMF et laisse 10 minutes sous agitation à 5°C. On ajoute ensuite une solution de 10 g de composé obtenu à l'étape précédente dans 120 ml de DMF et laisse 4 heures sous agitation en laissant remonter la température à TA. On verse le mélange réactionnel dans l'eau, extrait au DCM, lave la phase organique par une solution saturée de NaCl, sèche sur MgSO₄ et évapore le solvant sous vide. On chromatographie le résidu sur gel de silice en éluant par le mélange cyclohexane/AcOEt (50/50 ; v/v). On obtient 5 g du composé attendu.

### Préparation 1.8

N-[3-(6,7-Dichloro-2-méthyl-1*H*-indol-1-yl)propyl]méthanesulfonamide.
(II) : R₁ = -NHSO₂CH₃ ; R₂ = -CH₃ ; R₄ = 6-Cl ; R₅ = 7-Cl.

On prépare ce composé selon le procédé décrit à l'étape C de l'Exemple 72 dans WO 02/42269.

### Préparation 1.9

N-[3-(5,7-Dichloro-2-méthyl-1*H*-indol-1-yl)propyl]méthanesulfonamide.
(II) : R₁ = -NHSO₂CH₃ ; R₂ = -CH₃ ; R₄ = 5-Cl ; R₅ = 7-Cl.

On prépare ce composé selon les procédés décrits aux étapes A, B, C et D de la Préparation 1.7.

### Préparation 1.10

N-[3-(7-Chloro-2-méthyl-1*H*-indol-1-yl)propyl]-1,1,1-trifluorométhane sulfonamide.
(II) : R₁ = -NHSO₂CF₃ ; R₂ = -CH₃ ; R₄ = H ; R₅ = 7-Cl.

On prépare ce composé décrit dans le TABLEAU 3 à la Préparation 3.19 dans WO 02/42269 selon les méthodes citées.

### Préparation 1.11

N'-[3-(6,7-Dichloro-2-méthyl-1*H*-indol-1-yl)propyl]-N,N-diméthylsulfamide.
(II) : R₁ = -NHSO₂N(CH₃)₂ ; R₂ = -CH₃ ; R₄ = 6-Cl ; R₅ = 7-Cl.

### A) 6,7-Dichloro-1-(3-iodopropyl)-2-méthyl-1H-indole.

On prépare ce composé selon le mode opératoire décrit à l'étape B de l'Exemple 72 dans WO 02/42269.

### B) N'-[3-(6,7-Dichloro-2-méthyl-1H-indol-1-yl)propyl]-N,N-diméthylsulfamide.

On refroidit à 5°C un mélange de 2,8 g d'hydrure de sodium à 60 % dans l'huile dans 200 ml de DMF, ajoute, goutte à goutte, une solution de 8,68 g de N,N-diméthylsulfamide dans 100 ml de DMF et laisse 10 minutes sous agitation à 5°C. On ajoute ensuite une solution de 6,45 g du composé de l'étape précédente dans 100 ml de DMF et laisse 4 heures sous agitation en laissant remonter la température à TA. On verse le mélange réactionnel dans l'eau, extrait au DCM, lave la phase organique à l'eau, par une solution saturée de NaCl, sèche sur MgSO₄ et évapore le solvant sous vide. On chromatographie le résidu sur gel de silice en éluant par le mélange cyclohexane/AcOEt (70/30 ; v/v). On obtient 4,05 g du composé attendu.

### Préparation 1.12

7-Chloro-2-méthyl-1-[3-(méthylthio)propyl]-1*H*-indole.
(II) : R₁ = -S-CH₃ ; R₂ = -CH₃ ; R₄ = H ; R₅ = 7-Cl.

On prépare ce composé selon le mode opératoire décrit à l'étape B de l'Exemple 1 dans WO 02/42269.

### Préparation 1.13

6,7-Dichloro-1-[3-(méthylthio)propyl]-1*H*-indole.
(II) : R₁ = -S-CH₃ ; R₂ = H ; R₄ = 6-Cl ; R₅ = 7-Cl.

A une solution de 46,8 g du composé obtenu à l'étape C de la Préparation 1.7 dans 700 ml d'éthanol on ajoute 18,5 g de méthanethiolate de sodium et chauffe à reflux pendant une nuit. On verse le mélange réactionnel dans une solution de NaOH à 10 %, extrait à l'éther, lave la phase organique par une solution de NaOH à 10 %, à l'eau, par une solution saturée de NaCl, sèche sur MgSO₄ et évapore le solvant sous vide. On chromatographie le résidu sur gel de silice en éluant par le mélange cyclohexane/AcOEt (95/5 ; v/v). On obtient 28,3 g du composé attendu.

### Préparation 1.14

6,7-Dichloro-2-méthyl-1-[3-(méthylthio)propyl]-1*H*-indole.
(II) : R₁ = -S-CH₃ ; R₂ = -CH₃ ; R₄ = 6-Cl ; R₅ = 7-Cl.

### A) 6,7-Dichloro-1-(3-iodopropyl)-2-méthyl-1H-indole.

On prépare ce composé selon le mode opératoire décrit à l'étape B de l'Exemple 72 dans WO 02/42269.

### B) 6,7-Dichloro-2-méthyl-1-[3-(méthylthio)propyl]-1H-indole.

On chauffe à reflux pendant 24 heures un mélange de 12,6 g du composé obtenu à l'étape précédente et 4,79 g de méthanethiolate de sodium dans 250 ml d'éthanol. On verse le mélange réactionnel dans une solution de NaOH à 10 %, extrait à l'éther, lave la phase organique à l'eau, par une solution saturée de NaCl, sèche sur MgSO₄ et évapore le solvant sous vide. On chromatographie le résidu sur gel de silice en éluant au cyclohexane. On obtient 3,1 g du composé attendu.

### Préparation 1.15

N-[3-(7-méthoxy-2-méthyl-1*H*-indol-1-yl)propyl]méthanesulfonamide.
(II) : R₁ = -NHSO₂CH₃ ; R₂ = -CH₃ ; R₄ = H ; R₅ = 7-OCH₃.

### A) 7-Méthoxy-2-méthyl-1H-indole.

On refroidit à -40°C, sous atmosphère d'azote, 800 ml d'une solution 0,5 M de bromure d'isopropénylmagnésium dans le THF puis ajoute goutte à goutte, une solution de 20,4 g de 2-méthoxynitrobenzène dans 125 ml de THF et laisse 1 heure sous agitation en laissant remonter la température à 0°C. On verse le mélange réactionnel dans une solution saturée de NH₄Cl, extrait à l'éther, lave la phase organique par une solution saturée de NaCl, sèche sur MgSO₄ et évapore le solvant sous vide. On chromatographie le résidu sur gel de silice en éluant au cyclohexane puis par le mélange cyclohexane/AcOEt (90/10 ; v/v). On obtient 2,45 g du composé attendu sous forme d'huile.

### B) 1-(3-Chloropropyl)-7-méthoxy-2-méthyl-1H-indole.

A une solution de 2,45 g du composé de l'étape précédente dans 100 ml de toluène, on ajoute 3,41 g de KOH pilée et 0,245 g de tétrabutylammonium hydrogénosulfate puis chauffe à reflux pendant 20 minutes. On ajoute ensuite 6 ml de 1-bromo-3-chloropropane et poursuit le reflux pendant 1 heure. Après refroidissement à TA, on verse le mélange réactionnel dans l'eau, extrait au toluène, lave la phase organique par une solution saturée de NaCl, sèche sur MgSO₄ et évapore le solvant sous vide. On obtient 5,15 g du composé attendu sous forme d'huile.

### C) 1-(3-Iodopropyl)-7-méthoxy-2-méthyl-1H-indole.

A une solution de 5,05 g du composé de l'étape précédente dans 140 ml d'acétonitrile on ajoute 22,3 g de NaI et chauffe à reflux pendant une nuit. On concentre le mélange réactionnel sous vide, reprend le résidu à l'eau, extrait au toluène, lave la phase organique par une solution saturée de NaCl, sèche sur MgSO₄ et évapore le solvant sous vide. On obtient 6,3 g du composé attendu.

### D) N-[3-(7-méthoxy-2-méthyl-1-H-indol-1-yl)propyl]méthanesulfonamide.

On refroidit à 5°C un mélange de 3,04 g d'hydrure de sodium à 60% dans l'huile dans 200 ml de DMF, ajoute une solution de 7,2 g de méthanesulfonamide dans 90 ml de DMF et laisse 5 minutes sous agitation à 5°C. On ajoute ensuite une solution de 6,25 g du composé de l'étape précédente dans 90 ml de DMF et laisse une nuit sous agitation en laissant remonter la température à TA. On verse le mélange réactionnel dans l'eau, extrait au DCM, lave la phase organique par une solution saturée de NaCl, sèche sur MgSO₄ et évapore le solvant sous vide. On chromatographie le résidu sur gel de silice en éluant par le mélange cyclohexane/AcOEt de (80/20;v/v) à (40/60;v/v). On obtient 1,85 g du composé attendu.

### Préparation 1.16

N-[3-(7-nitro-1*H*-indol-1-yl)propyl]méthanesulfonamide.
(II) : R₁ = -NHSO₂CH₃ ; R₂ = H ; R₄ = H ; R₅ = 7-NO₂.

On prépare ce composé selon les procédés décrits aux étapes B, C et D de la Préparation 1.15 à partir de 7-nitro-1*H*-indole.

### Préparation 1.17

N-[3-(7-fluoro-2-méthyl-1*H*-indol-1-yl)propyl]trifluorométhanesulfonamide.
(II) : R₁ = -NHSO₂CF₃ ; R₂ = CH₃ ; R₄ = H ; R₅ = 7-F.

On refroidit à 5°C un mélange de 4,14 g d'hydrure de sodium à 60% dans l'huile dans 200 ml de DMF, ajoute une solution de 15,42 g de trifluorométhanesulfonamide dans 150 ml de DMF et laisse 5 minutes sous agitation à 5°C. On ajoute ensuite une solution de 8,2 g du composé de l'étape C de la Préparation 1.5 dans 100 ml de DMF et laisse une nuit sous agitation en laissant remonter la température à TA. On verse le mélange réactionnel dans l'eau, extrait au DCM, lave la phase organique par une solution saturée de NaCl, sèche sur MgSO₄ et évapore le solvant sous vide. On chromatographie le résidu sur gel de silice en éluant au cyclohexane puis par le mélange cyclohexane/AcOEt (80/20;v/v). On obtient 3,8 g du composé attendu.

### EXEMPLE 1 : Composé N° 1

### N-[3-[3-(3,3-Diméthylbutanoyl)-2-méthyl-1H-indol-1-yl]propyl]méthane sulfonamide.

On refroidit à -25°C sous atmosphère d'azote un mélange de 0,95 g du composé de la Préparation 1.1 et 0,99 ml de chlorure de 3,3-diméthylbutyryle dans 120 ml de DCM, ajoute 4 ml d'une solution 1,8 M de dichlorure d'éthylaluminium dans le toluène et laisse 5 heures sous agitation en laissant remonter la température à TA. On verse le mélange réactionnel dans l'eau, extrait au DCM, lave la phase organique par une solution d'HCl à 10 %, par une solution de NaOH à 10 %, par une solution saturée de NaCl, sèche sur MgSO₄ et évapore le solvant sous vide. On chromatographie le résidu sur gel de silice en éluant au DCM puis par le gradient du mélange DCM/MeOH de (100/0,5 ; v/v) à (100/2 ; v/v). On obtient 0,22 g du composé attendu, F = 139-142°C.

### EXEMPLE 2 : Composé N°40 et composé N°41 1-[7-Chloro-2-méthyl-1-[3-(méthylsulfinyl)propyl]-1H-indol-3-yl]-3,3-diméthylbutan-1-one et 1-[7-Chloro-2-méthyl-1-[3-(méthylsulfonyl)propyl]-1H-indol-3-yl]-3,3-diméthylbutan-1-one.

On refroidit à 5°C un mélange de 1,28 g d'acide 3-chloroperbenzoïque dans 72 ml de DCM, ajoute, goutte à goutte, une solution de 1,31 g du composé N° 52 dans 72 ml de DCM et laisse sous agitation en laissant remonter la température à TA. On verse le mélange réactionnel dans une solution de NaOH à 10 %, extrait au DCM, lave la phase organique à l'eau, par une solution de NaOH à 10 %, sèche sur MgSO₄ et évapore le solvant sous vide. Les deux composés obtenus sont séparés par chromatographie préparative selon les conditions suivantes :
- Appareillage : système HPLC préparatif Delta Prep 4000
- pompe WATERS LC PREP 4000 ;
- colonne PROCHROM à compression dynamique axiale : Longueur : 300 mm ; diamètre : 50 mm ;
- détecteur monolongueur d'onde TPS SPECTRA 100 ;

- Phase stationnaire : Kromasil C 18 10 µm
- Pression de compression : 50 bars ;

- Phase mobile :
éluant A : H₂O + TFA 0,1 %
éluant B : acétonitrile/H₂O (90/10 ; v/v) + TFA 0,1 %.

- Gradient :

| t(minute) | % A | % B |
|---|---|---|
| 0 | 15 | 85 |
| 50 | 09 | 91 |
| 60 | 09 | 91 |

- Débit : 124 ml/minute ;
- Détection UV : λ = 230 nm ; longueur de cellule : 3,5 mm. Conditions utilisées pour l'analyse HPLC :
- Appareillage : chaîne Waters avec logiciel Millennium ;
- Colonne : Uptisphère HDO 15 Q 5 5µm ; 150 x 4,6 mm ;
- Phase mobile :
éluant A : H₂O + TFA 0,1 %
éluant B : acétonitrile/H₂O (90/10 ; v/v) + TFA 0,1 %

- Gradient :

| t(minute) | % A | % B |
|---|---|---|
| 0 | 90 | 10 |
| 40 | 0 | 100 |
| 60 | 0 | 100 |

- Débit : 1 ml/minute ;
- Longueur d'onde : λ = 230 nm ;

Après séparation des composés ; on obtient :
- composé N° 53 : m = 0,257 g, F = 123°C dont la pureté sur phase inverse est de 99,8 % (tr = 31,13 mn) ;
- composé N° 54 : m = 0,358 g, F = 167°C dont la pureté sur phase inverse est de 98,3 % (tr = 34,95 mn).

### EXEMPLE 3 : Composé N°46 et Composé N°48

### 2-(1-Adamantyl)-1-[6,7-dichloro-2-méthyl-1-[3-(méthylsulfinyl)propyl]-1H-indol-3-yl]éthanone et 2-(1-adamantyl)-1-[6,7-dichloro-2-méthyl-1-[3-(méthylsulfonyl)propyl]-1H-indol-3-yl]éthanone.

On prépare ces deux composés selon le mode opératoire décrit à l'Exemple 3 à partir du composé N° 63. Les deux composés obtenus sont séparés par chromatographie préparative selon les mêmes conditions que celles décrites à l'Exemple 3. On obtient :
- composé N° 64 : m = 0,549 g, F = 116°C dont la pureté sur phase inverse est de 99,7 % (tr = 43,8 minutes),
- composé N° 65 : m = 0,462 g, F = 174°C dont la pureté sur phase inverse est de 98,7 % (tr = 46,1 minutes),

Le tableau qui suit illustre les structures chimiques et les propriétés physiques de quelques exemples de composés selon l'invention. Dans ce tableau :
- Me, Et représentent respectivement des groupes méthyle et éthyle :

**TABLEAU I**

| | | | | | | | |
|---|---|---|---|---|---|---|---|
| 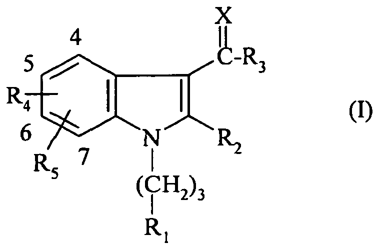 | | | | | | | |

| Composés N° | X | R₁ | R₂ | R₃ | R₄ | R₅ | F°C ; RMN |
|---|---|---|---|---|---|---|---|
| 1 | O | NH-SO₂ Me | Me | -CH₂-C(CH₃)₃ | H | H | 139-142 |
| 2 | O | NH-SO₂Me | Me | 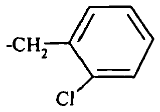 | H | H | 174-176 |
| 3 | O | NH-SO₂Me | Me | -C(CH₃)₃ | H | 7-Br | 89 |
| 4 | O | NH-SO₂Me | Me | -CH₂-C(CH₃)₃ | H | 7-Br | 93 |
| 5 | O | NH-SO₂Me | Me | 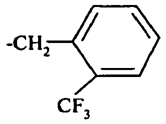 | H | 7-Br | 142 |
| 6 | O | NH-SO₂Me | Me | -CH₂-CH(CH₃)₂ | H | 7-Cl | 118-120 |
| 7 | O | NH-SO₂Me | Me | -CH₂-C(CH₃)₃ | H | 7-Cl | 113 |
| 8 | O | NH-SO₂Me | Me | 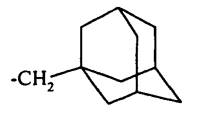 | H | 7-Cl | 55-60 |
| 9 | O | NH-SO₂Me | Me | 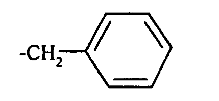 | H | 7-Cl | 159-161 |
| 10 | O | NH-SO₂Me | Me | 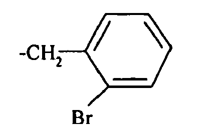 | H | 7-Cl | 149-152 |
| 11 | O | NH-SO₂Me | Me | 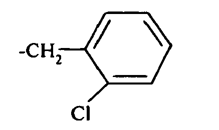 | H | 7-Cl | 170-171 |
| 12 | O | NH-SO₂Me | Me | 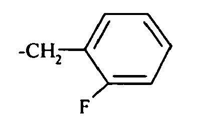 | H | 7-Cl | 160-164 |
| 13 | O | NH-SO₂Me | Me | 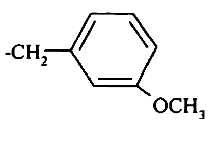 | H | 7-Cl | 119-122 |
| 14 | O | NH-SO₂Me | Me | 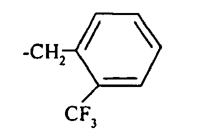 | H | 7-Cl | 139-141 RMN |
| 15 | O | NH-SO₂Me | Me | 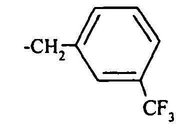 | H | 7-Cl | 106-108 |
| 16 | O | NH-SO₂Me | Me | 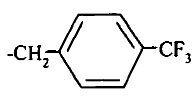 | H | 7-Cl | 136-138 |
| 17 | O | NH-SO₂Me | Me | 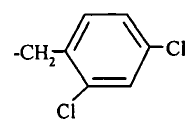 | H | 7-Cl | 141-144 |
| 18 | O | NH-SO₂Me | Me | 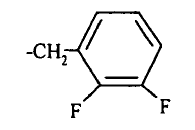 | H | 7-Cl | 145-147 |
| 19 | O | NH-SO₂Me | Me | 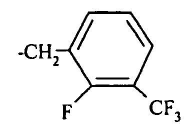 | H | 7-Cl | 143-146 |
| 20 | O | NH-SO₂Me | Me | 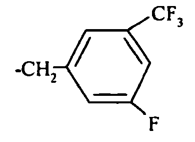 | H | 7-Cl | 111-115 |
| 21 | O | NH-SO₂Me | Me | 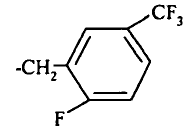 | H | 7-Cl | 120-124 |
| 22 | O | NH-SO₂Me | Me | 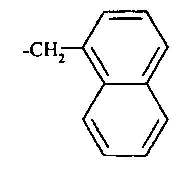 | H | 7-Cl | 128-130 |
| 23 | O | NH-SO₂Me | Me | 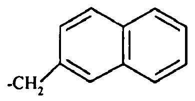 | H | 7-Cl | 163-165 |
| 24 | O | NH-SO₂Me | Me | 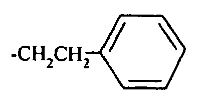 | H | 7-Cl | 137-140 |
| 25 | O | NH-SO₂Me | Me | 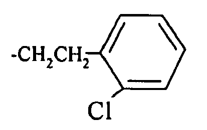 | H | 7-Cl | 127-129 |
| 26 | O | NH-SO₂Me | Me | 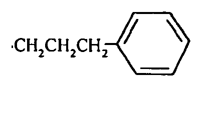 | H | 7-Cl | 127-130 |
| 27 | O | NH-SO₂Me | Me | 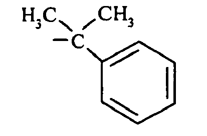 | H | 7-Cl | MH⁺=447 tr=9,61 |
| 28 | O | NH-SO₂Me | Me | 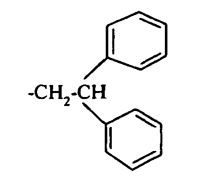 | H | 7-Cl | 129-132 |
| 29 | O | NH-SO₂Me | Et | 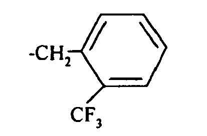 | H | 7-Cl | 107-109 |
| 30 | O | NH-SO₂Me | Me | 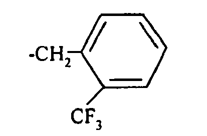 | H | 7-F | 112-115 |
| 31 | O | NH-SO₂Me | Me | -CH₂-C(CH₃)₃ | 6-CH₃ | 7-Br | 110-114 |
| 32 | O | NH-SO₂Me | Me | 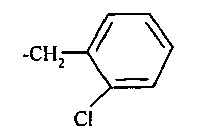 | 6-CH₃ | 7-Br | 179-181 |
| 33 | O | NH-SO₂Me | H | 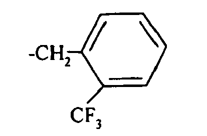 | 6-Cl | 7-Cl | 182-186 |
| 34 | O | NH-SO₂Me | Me | -CH₂-C(CH₃)₃ | 6-Cl | 7-Cl | 119-122 |
| 35 | O | NH-SO₂Me | Me | 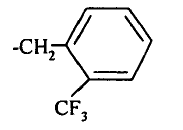 | 6-Cl | 7-Cl | 157-159 |
| 36 | O | NH-SO₂Me | Me | 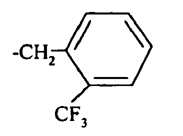 | 6-Cl | 7-Cl | 159-162 |
| 37 | O | NH-SO₂ CF3 | Me | 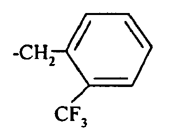 | H | 7-Cl | 127-130 |
| 38 | O | NHSO₂N(Me)₂ | Me | 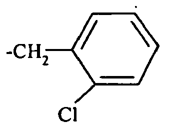 | 6-Cl | 7-Cl | 181 |
| 39 | O | -S-Me | Me | -CH₂-C(CH₃)₃ | H | 7-Cl | MH⁺=352 tr= 11,75 |
| 40 | O | -SO-Me | Me | -CH₂-C(CH₃)₃ | H | 7-Cl | 123 |
| 41 | O | -SO₂-Me | Me | -CH₂-C(CH₃)₃ | H | 7-Cl | 167 |
| 42 | O | -S-Me | Me | 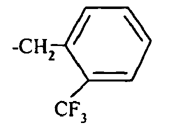 | H | 7-Cl | 76-80 |
| 43 | O | -SO₂-Me | Me | 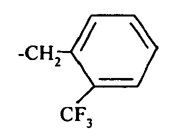 | H | 7-Cl | 140-142 |
| 44 | O | -S-Me | H | 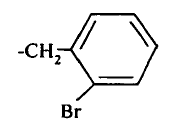 | 6-Cl | 7-Cl | 138-140 |
| 45 | O | -SO₂-Me | H | 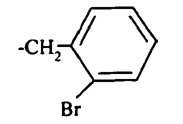 | 6-Cl | 7-Cl | 184 |
| 46 | O | -S-Me | Me | 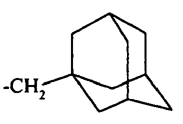 | 6-Cl | 7-Cl | 108 |
| 47 | O | -SO-Me | Me | 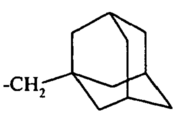 | 6-Cl | 7-Cl | 116 |
| 48 | O | -SO₂-Me | Me | 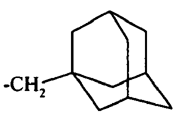 | 6-Cl | 7-Cl | 174 |
| 49 | O | -NH-SO₂-Me | Me | 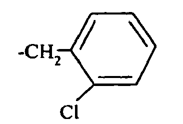 | H | 7-Br | 178-180 |
| 50 | O | -NH-SO₂-Me | Me | 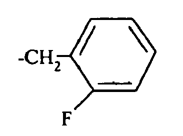 | H | 7-Br | 168-169 |
| 51 | O | -NH-SO₂-Me | Me | 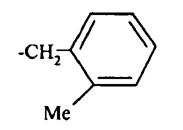 | H | 7-Br | 184-186 |
| 52 | O | -NH-SO₂-Me | Me | 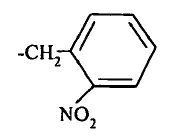 | H | 7-Br | 181 |
| 53 | O | -NH-SO₂-Me | Me | 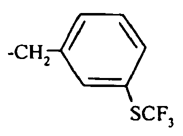 | H | 7-Br | 118-119 |
| 54 | O | -NH-SO₂-Me | Me | 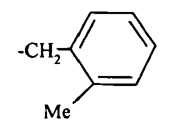 | H | 7-Cl | 178-180 |
| 55 | O | -NH-SO₂-Me | Me | 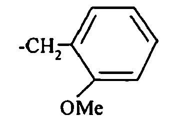 | H | 7-Cl | 126-128 |
| 56 | O | -NH-SO₂-Me | Me | 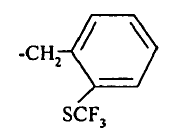 | H | 7-Cl | 142-144 |
| 57 | O | -NH-SO₂-Me | Me | 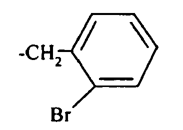 | H | 7-F | 155-157 |
| 58 | O | -NH-SO₂-Me | Me | 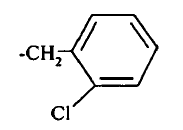 | H | 7-F | 143-146 |
| 59 | O | -NH-SO₂-Me | Me | 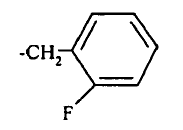 | H | 7-F | 145-147 |
| 60 | O | -NH-SO₂-Me | Me | 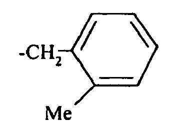 | H | 7-F | 56-58 |
| 61 | O | -NH-SO₂-Me | Me | 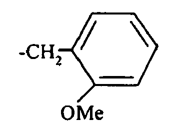 | H | 7-F | 113-116 |
| 62 | O | -NH-SO₂-Me | Me | 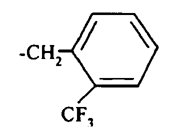 | 6-Me | 7-Br | 149-152 |
| 63 | O | -NH-SO₂-Me | Me | 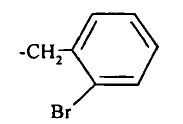 | H | 7-OMe | 164-167 |
| 64 | O | -NH-SO₂-Me | Me | 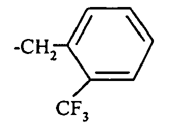 | H | 7-OMe | 182-184 |
| 65 | O | -NH-SO₂-Me | H | 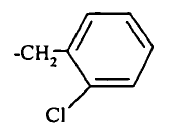 | H | 7-NO₂ | 177-178 |
| 66 | O | -NH-SO₂-M | H | 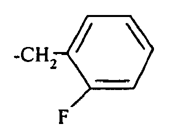 | H | 7-NO₂ | 177-178 |
| 67 | O | -NH-SO₂-Me | H | 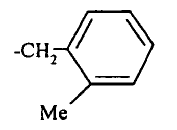 | H | 7-NO₂ | 135-137 |
| 68 | O | -NH-SO₂-M | H | 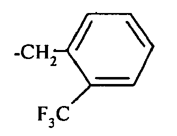 | H | 7-NO₂ | 162-165 |
| 69 | O | -NH-SO₂-M | H | 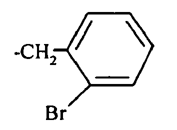 | H | 7-NO₂ | 184-187 |
| 70 | O | -NH-SO₂-M | H | 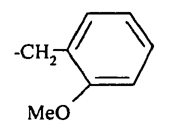 | H | 7-NO₂ | 178-179 |
| 71 | O | -NH-SO₂-M | H | 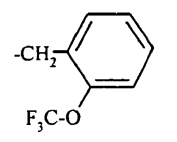 | H | 7-NO₂ | 168-170 |
| 72 | O | -NH-SO₂-M | H | 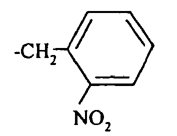 | H | 7-NO₂ | 156-159 |
| 73 | O | -NH-SO₂-Me | Me | 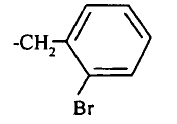 | H | 7-Br | 187-189 |
| 74 | O | -NH-SO₂-M | Me | 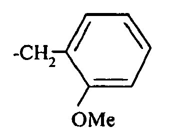 | H | 7-Br | 67-70 |
| 75 | O | -NH-SO₂-M | Me | 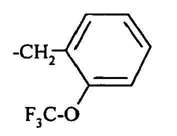 | H | 7-Br | 156-159 |
| 76 | O | -NH-SO₂-Me | Me | 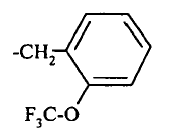 | H | 7-F | 119-122 |
| 77 | O | -NH-SO₂-Me | Me | 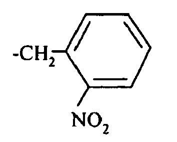 | H | 7-F | 65-67 |
| 78 | O | -NH-SO₂-CF₃ | Me | 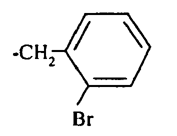 | H | 7-Cl | 139-141 |
| 79 | O | -NH-SO₂-CF₃ | Me | 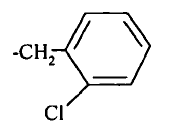 | H | 7-Cl | 135-138 |
| 80 | O | -NH-SO₂-CF₃ | Me | 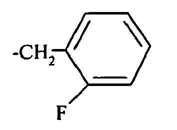 | H | 7-Cl | 141-144 |
| 81 | O | -NH-SO₂-CF₃ | Me | 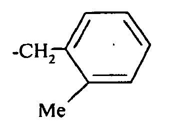 | H | 7-Cl | 135-137 |
| 82 | O | -NH-SO₂-CF₃ | Me | 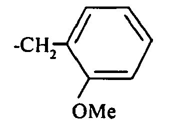 | H | 7-Cl | 178-180 |
| 83 | O | -NH-SO₂-CF₃ | Me | 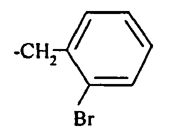 | H | 7-F | 63-65 |
| 84 | O | -NH-SO₂-CF₃ | Me | 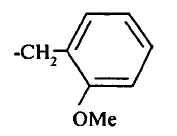 | H | 7-F | 152-155 |
| 85 | O | -NH-SO₂-CF₃ | Me | 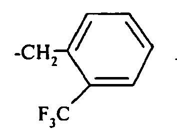 | H | 7-F | 151-154 |
| 86 | O | -NH-SO₂-Me | Me | 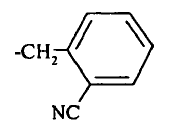 | H | 7-Br | 211-213 |

Composé N° 18 : RMN¹H : DMSO-d₆ : δ(ppm) : 1,95 : mt : 2H ; 2,75 : s : 3H ; 2,95 : s : 3H ; 3,15 : qd : 2H ; 4,4 -4,8 : s+mt : 4H ; 7,15-7,4 : mt : 3H ; 7,5 : mt : 2H ; 7,6-7,8 : mt : 2H ; 8,1 : d : 1 H.

Les composés selon l'invention ont montré une bonne affinité *in vitro* pour les récepteurs aux cannabinoïdes (CB₂) et une affinité *in vitro* nettement plus faible pour les récepteurs aux cannabinoïdes (CB₁) qu'il s'agisse de récepteurs humains ou de récepteurs de rongeur. Les essais de liaison par affinité (binding) ont été réalisés selon les conditions expérimentales décrites par M. Rinaldi-Carmona dans J. Pharmacol. Exp. Therap. 1998, 287, 644-650, avec des membranes issues de tissus de rongeur et de lignée cellulaires dans lesquelles les récepteurs CB₁ (Matsuda et al., Nature 1990, 346, 561-564) et CB₂ (Munro et al., Nature 1993, 365, 61-65) ont été exprimés. L'affinité *in vitro* aux récepteurs cannabinoïdes, est exprimée sous forme de CI₅₀ (concentration causant une inhibition de 50% de la liaison spécifique du contrôle).

Pour les récepteurs humains, l'affinité in vitro aux cannabinoïdes CB₂ exprimée sous forme de CI₅₀ est inférieure à 500nM.

La nature agoniste des composés selon l'invention a été démontrée par les résultats obtenus dans les modèles de l'inhibition de l'adénylate-cyclase comme décrit dans M. Rinaldi-Carmona et al., J. Pharmacol. Exp. Ther. 1996, 278, 871-878 et 1998, 284, 644-650 et M. Bouaboula et al. J. Biol Chem., 1997, 272, 22330-122339.

Les composés de la présente invention sont notamment des principes actifs de compositions pharmaceutiques dont la toxicité est compatible avec leur utilisation en tant que médicaments.

Selon un de ses aspects, la présente invention concerne l'utilisation d'un composé de formule (I) ou d'un de ses sels ou solvats, pharmaceutiquement acceptable pour la préparation de médicaments destinés à prévenir ou à traiter toute pathologie humaine et / ou à usage vétérinaire dans laquelle les récepteurs aux cannabinoïdes CB₂ sont impliqués.

On peut par exemple citer les maladies ou affections suivantes :
les désordres du système immunitaire, notamment les maladies autoimmunes : psoriasis, lupus érythémateux, maladies du tissu conjonctif ou connectivites, syndrome de Sjögrer's, spondylarthrite ankylosante, arthrite rhumatoïde, arthrite réactionnelle, spondylarthrite indifférenciée, maladie de Behcet's, anémies autoimmunes hémolytiques, sclérose en plaques, sclérose latérale amyotrophique, amyloses, le rejet de greffe, les maladies affectant la lignée plasmocytaire ; les maladies allergiques: hypersensibilité retardée ou immédiate, rhinite allergique, dermatite de contact, conjonctivite allergique ; les maladies infectieuses parasitaire, virale ou bactérienne : SIDA, méningites ; l'amylose, les maladies affectant les lignées du système lympho-hématopoîétique ; les maladies chroniques du foie d'origine alcoolique, virale et toxique ainsi que stéato hépatites d'origine non alcoolique et le cancer primaire du foie ; les maladies inflammatoires, notamment les maladies articulaires : arthrite, arthrite rhumatoïde, ostéoarthrite, spondylite, goutte, vascularite, maladie de Crohn, maladie du colon irritable syndrome de colon irritable (en anglais IBD : inflammatory bowel disease et IBS : irritable bowel syndrome), pancréatite ; l'ostéoporose ; la douleur : les douleurs chroniques de type inflammatoire, les douleurs neuropathiques, les douleurs aiguës périphériques ; les affections oculaires : hypertension oculaire, glaucome ; les affections pulmonaires : maladies des voies respiratoires, asthme, bronchite chronique, obstruction chronique des voies respiratoires (en anglais COPD : chronic obstructive pulmonary disease), emphysème ; les maladies du système nerveux central et les maladies neurogénératives : syndrome de Tourette, maladie de Parkinson, maladie d'Alzheimer, démence sénile, chorée, chorée de Huntington, épilepsie, psychoses, dépression, lésions de la moelle épinière ; la migraine, les vertiges, les vomissements, les nausées en particulier ceux consécutifs à une chimiothérapie ; les maladies cardiovasculaires en particulier hypertension, artériosclérose, crise cardiaque, ischémie cardiaque ; l'ischémie rénale ; les cancers : les tumeurs bégnines de la peau, papillomes et tumeurs cancéreuses, les tumeurs de la prostate, les tumeurs cérébrales (glioblastomes, médullo-epithéliomes, médulloblastomes, neuroblastomes, tumeurs d'origine embryonnaires, astrocytomes, astroblastomes, épendymomes, oligodendrogliomes, tumeur du plexus, neuro-epithéliomes, tumeur de l'épiphyse, épendymoblastomes, neuroectodermique, méningiomes malins, sarcomatoses, mélanomes malins, schwannomes) ; les maladies gastro-intestinales ; l'obésité ; le diabète.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels ou solvats pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Ainsi, selon un autre de ses aspects, la présente invention concerne des compositions pharmaceutiques renfermant, en tant que principe actif, un composé de formule (I), ou l'un de ses sels ou solvats pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou hydrates ou solvats.

Les composés selon l'invention pourront également être utilisés pour la préparation de compositions à usage vétérinaire.

De plus, les composés selon l'invention, tel quel ou sous forme radiomarquée peuvent être utilisés comme outils pharmacologiques chez l'homme ou chez l'animal, pour la détection et le marquage des récepteurs aux cannabinoïdes CB₂.

## Revendications

1. Composé répondant à la formule (I) : 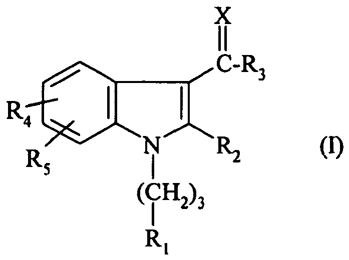 dans laquelle :
- X représente un atome d'oxygène;
- R₁ représente un groupe choisi parmi -N(R₇)SO₂R₈, -N(R₇)SO₂NR₉R₁₀, - SR₈, -SOR₈, -SO₂R₈, -SO₂NR₉R₁₀ ;
- R₂ représente un atome d'hydrogène ou un (C₁-C₄)alkyle ;
- R₃ représente :
. un (C₁-C₆)alkyle non substitué ou substitué par :
a) un ou plusieurs atomes d'halogène ;
b) un carbocycle non aromatique en (C₃-C₁₂), saturé ou insaturé, non substitué ou substitué une ou plusieurs fois par un (C₁-C₄)alkyle ;
c) un phényle non substitué ou substitué une ou plusieurs fois par des substituants choisis indépendamment parmi un atome d'halogène, un groupe Alk, un groupe OAlk, un groupe -S(O)ₙAlk, un nitro, un cyano ;
d) un naphtyle non substitué ou substitué une ou plusieurs fois par des substituants choisis indépendamment parmi un atome d'halogène, un (C₁-C₄)alkyle, un (C₁-C₄)alcoxy, un radical trifluorométhyle ;
. un benzhydrylméthyle ;
- R₄ et R₅ représentent chacun indépendamment un atome d'hydrogène, un atome d'halogène, un (C₁-C₄)alkyle, un (C₁-C₄)alcoxy, un radical trifluorométhyle, un nitro;
- R₇ représente un atome d'hydrogène ou un (C₁-C₄)alkyle
- R₈ représente un (C₁-C₄)alkyle ou un radical trifluorométhyle ;
- R₉ et R₁₀ représentent chacun indépendamment un atome d'hydrogène ou un (C₁-C₄)alkyle ;
- n représente 0, 1 ou 2;
- Alk représente un (C₁-C₄) alkyle non substitué ou substitué une ou plusieurs fois par un atome de fluor ;
ainsi que ses hydrates ou ses solvats.

2. Composé de formule (IC) selon la revendication 1 dans laquelle :
- X représente un atome d'oxygène ;
- R₁ représente un groupe choisi parmi -NHSO₂CH₃, -NHSO₂CF₃,
- NHSO₂N(CH₃)₂, -S-CH₃, SO-CH₃, -SO₂-CH₃ ;
- R₂ représente un atome d'hydrogène, un méthyle, un éthyle ;
- R₃ représente :
. un *tert*-butyle, un isobutyle, un 2,2-diméthylpropyle, un adamantylméthyle, un phénylméthyle, un (2-bromophényl)méthyle, un (2-chlorophényl)méthyle, un (2-fluorophényl)méthyle, un (3-méthoxyphényl)méthyle, un [2-(trifluorométhyl) phényl]méthyle, un [3-(trifluorométhyl)phényl]méthyle, un [4-(trifluorométhyl) phényl]méthyle, un (2,4-dichlorophényl)méthyle, un (2,3-difluorophényl) méthyle, un [2-fluoro-3-(trifluorométhyl)phényl]méthyle, un [2-fluoro-5-(trifluorométhyl)phényl]méthyle, un [3-fluoro-5-(trifluorométhyl)phényl] méthyle, un 1-méthyl-1-phényléthyle, un 2-phényléthyle, un 2-(2-chlorophényl) éthyle, un 3-phénylpropyle, un naphtylméthyle, un (2-méthylphényl)méthyle, un (2-nitrophényl)méthyle, un [3-(trifluorométhylthio)phényl]méthyle, un (2-méthoxyphényl)méthyle, un [2-(trifluorométhylthio)phényl]méthyle, un [2-(trifluorométhoxy)phényl]méthyle, un (2-cyanophényl)méthyle ;
. un benzhydrylméthyle ;
- R₄ représente un atome d'hydrogène ou est en position -6- de l'indole et représente un atome de chlore ou un méthyle ;
- R₅ représente un atome d'hydrogène ou est en position -7- de l'indole et représente un atome de brome, de chlore ou de fluor, un méthoxy, un nitro ; ainsi que ses hydrates ou ses solvats.

3. Composé de formule (I) selon la revendication 1 choisi parmi :
- N-[3-[7-bromo-3-(3,3-diméthylbutanoyl)-2-méthyl-1*H*-indol-1-yl]propyl]méthanesulfonamide ;
- N-[3-[7-bromo-2-méthyl-3-[[2-(trifluorométhyl)phényl]acétyl]-1*H*-indol-1-yl]propyl]méthanesulfonamide ;
- N-[3-[7-chloro-3-(3,3-diméthylbutanoyl)-2-méthyl-1*H*-indo1-1-yl]propyl]méthanesulfonamide ;
- N-[3-[3-(1-adamantylacétyl)-7-chloro-2-méthyl-1*H*-indol-1-yl]propyl]méthanesulfonamide ;
- N-[3-[3-[(2-bromophényl)acétyl]-7-chloro-2-méthyl-1*H*-indol-1-yl]propyl]méthanesulfonamide ;
- N-[3-[7-chloro-3-[(2-chlorophényl)acétyl]-2-méthyl-1*H*-indol-1-yl]propyl]méthanesulfonamide ;
- N-[3-[7-chloro-2-méthyl-3-[[2-(trifluorométhyl)phényl]acétyl]-1*H*-indol-1-yl]propyl]méthanesulfonamide ;
- N-[3-[7-chloro-3-[(2,3-difluorophényl)acétyl]-2-méthyl-1*H*-indol-1-yl]propyl]méthanesulfonamide ;
- N-[3-[7-fluoro-2-méthyl-3-[[2-(trifluorométhyl)phényl]acétyl]-1*H*-indol-1-yl]propyl]méthanesulfonamide ;
- N-[3-[7-bromo-3-(3,3-diméthylbutanoyl)-2,6-diméthyl-1*H* indol-1-yl]propyl]méthanesulfonamide ;
- N-[3-[7-bromo-3-[(2-chlorophényl)acétyl]-2,6-diméthyl-1*H*-indol-1-yl]propyl]méthanesulfonamide ;
- 1-[7-chloro-2-méthyl-1-[3-(méthylthio)propyl]-1*H*-indol-3-yl)-2-[2-(trifluorométhyl)phényl]éthanone ; N-[3-[7-bromo-3-[(2-chlorophényl)acétyl]-2-méthyl-1*H*-indol-1-yl]propyl]méthanesulfonamide ;
- N-[3-[7-bromo-3-[(2-fluorophényl)acétyl]-2-méthyl-1*H*-indol-1-yl]propyl]méthanesulfonamide ;
- N-[3-[7-bromo-2-méthyl-3-[(2-méthylphényl)acétyl]-1*H*-indol-1-l]propyl]méthanesulfonamide ;
- N-[3-[7-bromo-2-méthyl-3-[(2-nitrophényl)acétyl]-1*H*-indol-1-yl]propyl]méthanesulfonamide ;
- N-[3-[7-chloro-2-méthyl-3-[(2-méthylphényl)acétyl]-1*H*-indol-1-yl]propyl]méthanesulfonamide ;
- N-[3-[7-chloro-3-[(2-méthoxyphényl)acétyl]-2-méthyl-1*H*-indol-1-yl]propyl]méthanesulfonamide ;
- N-[3-[7-chloro-2-méthyl-3-[[2-[(trifluorométhyl)thio]phényl]acétyl]-1*H*-indol-1-yl]propyl]méthanesulfonamide ;
- N-[3-[3-[(2-bromophényl)acétyl]-7-fluoro-2-méthyl-1*H*-indol-1-yl]propyl]méthanesulfonamide ;
- N-[3-[3-[(2-chlorophényl)acétyl]-7-fluoro-2-méthyl-1*H*-indol-1-yl]propyl]méthanesulfonamide ;
- N-[3-[7-fluoro-3-[(2-fluorophényl)acétyl]-2-méthyl-1*H*-indol-1-yl]propyl]méthanesulfonamide ;
- N-[3-[7-fluoro-2-méthyl-3-[(2-méthylphényl)acétyl]-1*H*-indol-1-yl]propyl]méthanesulfonamide ;
- N-[3-[7-fluoro-3-[(2-méthoxyphényl)acétyl]-2-méthyl-1*H*-indol-1-yl]propyl]méthanesulfonamide ;
- N-[3-[7-bromo-2,6-diméthyl-3-[[2-(trifluorométhyl)phényl]acétyl]-1*H*-indol-1-yl]propyl]méthanesulfonamide ;
- N-[3-[3-[(2-bromophényl)acétyl]-7-méthoxy-2-méthyl-1*H*-indol-1-yl]propyl]méthanesulfonamide ;
- N-[3-(7-méthoxy-2-méthyl-3-[[2-(trifluorométhyl)phényl]acétyl]-1*H*-indol-1-yl]propyl]méthanesulfonamide ;
- N-[3-[3[(2-chlorophényl)acétyl]-7-nitro-1*H*-indol-1-yl]propyl]méthanesulfonamide ;
- N-[3-[3[(2-fluorophényl)acétyl]-7-nitro-1*H* indol-1-yl]propyl]méthanesulfonamide ;
- N-[3-[3[(2-méthylphényl)acétyl]-7-nitro-1*H*-indol-1-yl]propyl]méthanesulfonamide ;
- N-[3-(7-nitro-3-[[2-trifluorométhyl)phényl)acétyl]-1*H*-indol-1-yl]propyl]méthanesulfonamide ;
- N-[3-[3-[(2-bromophényl)acétyl]-7-nitro-1*H*-indol-1-yl]propyl]méthanesulfonamide ;
- N-[3-[3-[(2-methoxyphényl)acétyl]-7-nitro-1*H*-indol-1-yl]propyl]méthanesulfonamide ;
- N-[3-[7-nitro-3-[[(2-(trifluorométhoxy)phényl)acétyl]-1*H*-indol-1-yl]propyl]méthanesulfonamide ;
- N-[3-[7-nitro-3-[[(2-(nitrophényl)acétyl]-1*H*-indol-1-yl]propyl]méthanesulfonamide ;
- N-[3-[7-bromo-3-[(2-(bromophényl)acétyl]-2-méthyl-1*H*-indol-1-yl]propyl]méthanesulfonamide ;
- N-[3-[7-bromo-3-[(2-(méthoxyphényl)acétyl]-2-méthyl-1*H*-indol-1-yl]propyl]méthanesulfonamide ;
- N-[3-[7-bromo-2-méthyl-3-[[2-(trifluorométhoxy)phényl]acétyl]-1*H*-indol-1-yl]propyl]méthanesulfonamide ;
- N-[3-[7-fluoro-2-méthyl-3-[[2-(trifluorométhoxy)phényl]acétyl]-1*H*-indol-1-yl]propyl]méthanesulfonamide ;
- N-[3-[7-fluoro-2-méthyl-3-[(2-nitrophényl)acétyl]-1*H*-indol-1-yl]propyl]méthanesulfonamide;
- 1,1,1-trifluoro-N-[3-[7-fluoro-2-méthyl-3-[(2-trifluorométhyl)phényl)acétyl]-1*H*-indol-1-yl]propyl]méthanesulfonamide.
- 2-(1-adamantyl)-1-[6,7-dichloro-2-méthyl-1-[3-(méthylthio)propyl]-1*H*-indol-3-yl]éthanone ;
- 2-(1-adamantyl)-1-[6,7-dichloro-2-méthyl-1-[3-(méthylsulfinyl)propyl]-1*H-*indol-3-yl]éthanone ;
ainsi que ses hydrates ou ses solvats.

4. Procédé de préparation des composés de formule (I) selon la revendication 1 dans laquelle X représente un atome d'oxygène **caractérisé en ce que** :
on fait réagir, en présence d'un acide de Lewis, un composé de formule : 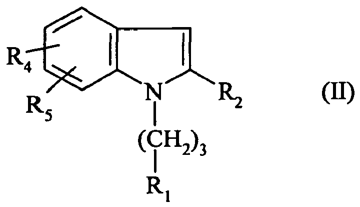
dans laquelle R₁, R₂, R₄ et R₅ sont tels que définis pour un composé de formule (I) à la revendication 1, avec un halogénure d'acide de formule : 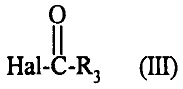 dans laquelle R₃ est tel que défini pour un composé de formule (I) à la revendication 1 et Hal représente un atome d'halogène.

5. Médicament, **caractérisé en ce qu'**il contient un composé de formule (I) selon l'une quelconque des revendications 1 à 3, ou encore un hydrate ou un solvat du composé de formule (I).

6. Composition pharmaceutique, **caractérisé en ce qu'**elle comprend un composé de formule (I) selon l'une quelconque des revendications 1 à 3, un hydrate ou un solvat de ce composé, ainsi qu'au moins un excipient pharmaceutiquement acceptable.

7. Utilisation d'un composé de formule (I) selon l'une quelconque des revendications 1 à 3 pour la préparation d'un médicament pour le traitement et la prévention des maladies auto-immunes, des maladies allergiques, des maladies infectieuses, des maladies neurogénératives, des maladies cardiovasculaires, des cancers, des maladies gastro-intestinales, de l'obésité, du diabète.

## Claims

1. Compound corresponding to the formula (I): 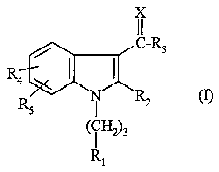 in which:
- X represents an oxygen atom;
- R₁ represents a group chosen from -N(R₇)SO₂R₈,
- N(R₇)SO₂NR₉R₁₀, -SR₈, -SOR₈, -SO₂R₈ or -SO₂NR₉R₁₀;
- R₂ represents a hydrogen atom or a (C₁-C₄)alkyl;
- R₃ represents:
. a (C₁-C₆)alkyl which is unsubstituted or substituted by:
a) one or more halogen atoms;
b) a nonaromatic C₃-C₁₂ carbocycle which is saturated or unsaturated and unsubstituted or substituted one or more times by a (C₁-C₄)alkyl;
c) a phenyl which is unsubstituted or substituted one or more times by substituents chosen independently from a halogen atom, an Alk group, an OAlk group, an -S(O)ₙ Alk group, a nitro or a cyano;
d) a naphthyl which is unsubstituted or substituted one or more times by substituents chosen independently from a halogen atom, a (C₁-C₄)alkyl, a (C₁-C₄)alkoxy or a trifluoromethyl radical;
. a benzhydrylmethyl;
- R₄ and R₅ each independently represent a hydrogen atom, a halogen atom, a (C₁-C₄)alkyl, a (C₁-C₄)alkoxy, a trifluoromethyl radical or a nitro;
- R₇ represents a hydrogen atom or a (C₁-C₄)alkyl;
- R₈ represents a (C₁-C₄)alkyl or a trifluoromethyl radical;
- R₉ and R₁₀ each independently represent a hydrogen atom or a (C₁-C₄)alkyl;
- n represents 0, 1 or 2;
- Alk represents a (C₁-C₄)alkyl which is unsubstituted or substituted one or more times by a fluorine atom;
and the hydrates or solvates thereof.

2. Compound of formula (I) according to Claim 1 in which:
- X represents an oxygen atom;
- R₁ represents a group chosen from -NHSO₂CH₃, -NHSO₂CF₃,
- NHSO₂N(CH₃)₂, -S-CH₃, -SO-CH₃ or -SO₂-CH₃;
- R₂ represents a hydrogen atom, a methyl or an ethyl;
- R₃ represents:
. a *tert*-butyl, an isobutyl, a 2,2-dimethylpropyl, an adamantylmethyl, a phenylmethyl, a (2-bromophenyl)methyl, a (2-chlorophonyl)methyl, a (2-fluorophenyl)methyl, a (3-methoxyphenyl)methyl, a [2-(trifluoromethyl)phenyl]methyl, a [3-(trifluoromethyl)phenyl]methyl, a [4-(trifluoromethyl)phenyl]methyl, a (2,4-dichlorophenyl)methyl, a (2,3-difluorophenyl)methyl, a [2-fluoro-3-(trifluoromethyl)phenyl]methyl, a [2-fluoro-5-(trifluoromethyl)phenyl]methyl, a [3-fluoro-5-(trifluoromethyl)phenyl]methyl, a 1-methyl-1-phenylethyl, a 2-phenylethyl, a 2-(2-chlorophenyl)ethyl, a 3-phenylpropyl, a naphthylmethyl, a (2-methylphenyl)methyl, a (2-nitrophenyl)methyl, a [3-(trifluoromethylthio)phenyl]methyl, a (2-methoxyphenyl)methyl, a [2- (trifluoromethylthio)phenyl]methyl, a [2-(trifluoromethoxy)phenyl]methyl or a (2-cyanophenyl)methyl;
. a benzhydrylmethyl;
- R₄ represents a hydrogen atom or is in the 6-position of the indole and represents a chlorine atom or a methyl;
- R₅ represents a hydrogen atom or is in the 7-position of the indole and represents a bromine, chlorine or fluorine atom, a methoxy or a nitro;
and the hydrates or solvates thereof.

3. Compound of formula (I) according to Claim 1 chosen from:
- N-[3-[7-bromo-3-(3,3-dimethylbutanoyl)-2-methyl-1*H*-indol-1-yl]propyl]methanesulphonamide;
- N-[3-[7-bromo-2-methyl-3-[[2-(trifluoromethyl)phenyl]acetyl]-1*H-*indol-1-yl]propyl]methanesulphonamide;
- N-[3-[7-chloro-3-(3,3-dimethylbutanoyl)-2-methyl-1*H*-indol-1-yl]propyl]methanesulphonamide;
- N-[3-[3-(1-adamantylacetyl)-7-chloro-2-methyl-1*H*-indol-1-yl]propyl]methanesulphonamide;
- N-[3-[3-[(2-bromophenyl)acetyl]-7-chloro-2-methyl-1*H*-indol-1-yl]propyl]methanesulphonamide;
- N-[3-[7-chloro-3-[(2-chlorophenyl)acetyl]-2-methyl-1*H*-indol-1-yl]propyl]methanesulphonamide;
- N-[3-[7-chloro-2-methyl-3-[[2-(trifluoromethyl)phenyl]acetyl]-1*H-*indol-1-yl]propyl]methanesulphonamide;
- N-[3-[7-chloro-3-[(2,3-difluorophenyl)acetyl]-2-methyl-1*H*-indol-1-yl]propyl]methanesulphonamide;
- N-[3-[7-fluoro-2-methyl-3-[[2-(trifluoromethyl)phenyl]acetyl]-1*H-*indol-1-yl]propyl]methanesulphonamide;
- N-[3-[7-bromo-3-(3,3-dimethylbutanoyl)-2,6-dimethyl-1*H*-indol-1-yl]propyl]methanesulphonamide;
- N-[3-[7-bromo-3-[(2-chlorophenyl)acetyl]-2,6-dimethyl-1*H*-indol-1-yl]propyl]methanesulphonamide;
- 1-[7-chloro-2-methyl-1-[3-(methylthio)propyl]-1*H*-indol-3-yl)-2-[2-(trifluoromethyl)phenyl]ethanone;
- N-[3-[7-bromo-3-[(2-chlorophenyl)acetyl]-2-methyl-1*H*-indol-1-yl]propyl]methanesulphonamide;
- N-[3-[7-bromo-3-[(2-fluorophenyl)acetyl]-2-methyl-1*H*-indol-1-yl]propyl]methanesulphonamide;
- N-[3-[7-bromo-2-methyl-3-[(2-methylphenyl)acetyl]-1*H*-indol-1-yl]propyl]methanesulphonamide;
- N-[3-[7-bromo-2-methyl-3-[(2-nitrophenyl)acetyl]-1*H*-indol-1-yl]propyl]methanesulphonamide;
- N-[3-[7-chloro-2-methyl-3-[(2-methylphenyl)acetyl]-1*H*-indol-1-yl]propyl]methanesulphonamide;
- N-[3-[7-chloro-3-[(2-methoxyphenyl)acetyl]-2-methyl-1*H*-indol-1-yl]propyl]methanesulphonamide;
- N-[3-[7-chloro-2-methyl-3-[[2-[(trifluoromethyl)thio]phenyl]acetyl]-1*H*-indol-1-yl]propyl]methanesulphonamide;
- N-[3-[3-[(2-bromophenyl)acetyl]-7-fluoro-2-methyl-1*H*-indol-1-yl]propyl]methanesulphonamide;
- N-[3-[3-[(2-chlorophenyl)acetyl]-7-fluoro-2-methyl-1*H*-indol-1-yl]propyl]methanesulphonamide;
- N-[3-[7-fluoro-3-[(2-fluorophenyl)acetyl]-2-methyl-1*H*-indol-1-yl]propyl]methanesulphonamide;
- N-[3-[7-fluoro-2-methyl-3-[(2-methylphenyl)acetyl]-1*H*-indol-1-yl]propyl]methanesulphonamide;
- N-[3-[7-fluoro-3-[(2-methoxyphenyl)acetyl]-2-methy]-1*H*-indo]-1-yl]propyl]methanesulphonamide;
- N-[3-[7-bromo-2,6-dimethyl-3-[[2-(trifluoromethyl)phenyl]acetyl]-1*H-*indol-1-yl]propyl]methanesulphonamide;
- N-[3-[3-[(2-bromophenyl)acetyl]-7-methoxy-2-methyl-1*H*-indol-1-yl]propyl]methanesulphonamide;
- N-[3-[7-methoxy-2-methyl-3-[[2-(trifluoromethyl)phenyl]acetyl]-1*H-*indol-1-yl]propyl]methanesulphonamide;
- N-[3-[3-[(2-chlorophenyl)acetyl]-7-nitro-1*H*-indol-1-yl]propyl]methanesulphonamide;
- N-[3-[3-[(2-fluorophenyl)acetyl]-7-nitro-1*H*-indol-1-yl]propyl]methanesulphonamide;
- N-[3-[3-[(2-methylphenyl)acetyl]-7-nitro-1*H*-indol-1-yl]propyl]methanesulphonamide;
- N-[3-[7-nitro-3-[[2-(trifluoromethyl)phenyl]acetyl]-1*H*-indol-1-yl]propyl]methanesulphonamide;
- N-[3-[3-[(2-bromophenyl)acetyl]-7-nitro-1*H*-indol-1-yl]propyl]methanesulphonamide;
- N-[3-[3-[(2-methoxyphenyl)acetyl]-7-nitro-1*H*-indol-1-yl]propyl]methanesulphonamide;
- N-[3-[7-nitro-3-[[2-(trifluoromethoxy)phenyl]acetyl]-1*H*-indol-1-yl]propyl]methanesulphonamide;
- N-[3-[7-nitro-3-[(2-nitrophenyl)acetyl]-1*H*-indol-1-yl]propyl]methanesulphonamide;
- N-[3-[7-bromo-3-[(2-bromophenyl)acetyl]-2-methyl-1*H*-indol-1-yl]propyl]methanesulphonamide;
- N-[3-[7-bromo-3-[(2-methoxyphenyl)acetyl]-2-methyl-1*H*-indol-1-yl]propyl]methanesulphonamide;
- N-[3-[7-bromo-2-methyl-3-[[2-(trifluoromethoxy)phenyl]acetyl]-1*H-*indol-1-yl]propyl]methanesulphonamide;
- N-[3-[7-fluoro-2-methyl-3-[[2-(trifluoromethoxy)phenyl]acetyl]-1*H-*indol-1-yl]propyl]methanesulphonamide;
- N-[3-[7-fluoro-2-methyl-3-[(2-nitrophenyl)acetyl]-1*H*-indol-1-yl]propyl]methanesulphonamide;
- 1,1,1-trifluoro-N-[3-[7-fluoro-2-methyl-3-[[2-(trifluoromethyl)phenyl]acetyl]-1*H*-indol-1-yl]propyl]methanesulphonamide;
- 2-(1-adamantyl)-1-[6,7-dichloxo-2-methyl-1-[3-(methylthio)propyl]-1*H*-indol-3-yl]ethanone;
- 2-(1-adamantyl)-1-[6,7-dichloro-2-methyl-1-[3-(methylsulphinyl)propyl]-1*H*-indol-3-yl]ethanone;
and the hydrates or solvates thereof.

4. Process for the preparation of the compounds of formula (I) according to Claim 1 in which X represents an oxygen atom, **characterized in that**:
a compound of formula: 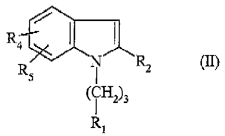
in which R₁, R₂, R₄ and R₅ are as defined for a compound of formula (I) in Claim 1, is reacted, in the presence of a Lewis acid, with an acid halide of formula: 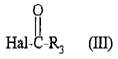 in which R₃ is as defined for a compound of formula (I) in Claim 1 and Hal represents a halogen atom.

5. Medicament, **characterized in that** it comprises a compound of formula (I) according to any one of Claims 1 to 3 or also a hydrate or a solvate of the compound of formula (I).

6. Pharmaceutical composition, **characterized in that** it comprises a compound of formula (I) according to any one of Claims 1 to 3, a hydrate or a solvate of this compound, and also at least one pharmaceutically acceptable excipient.

7. Use of a compound of formula (I) according to any one of Claims 1 to 3 in the preparation of a medicament for the treatment and prevention of autoimmune diseases, allergic diseases, infectious diseases, neurodegenerative diseases, cardiovascular diseases, cancers, gastrointestinal diseases, obesity and diabetes.

## Patentansprüche

1. Verbindung der Fomrel (I): 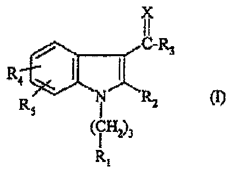 worin:
- X für ein Sauerstoffatom steht;
- R₁ für eine unter -N(R₇)SO₂R₈, -N(R₇)SO₂NR₉R₁₀,
- SR₈, -SOR₈, -SO₂R₈ und -SO₂NR₉R₁₀ ausgewählte Gruppe steht;
- R₂ für ein Wasserstoffatom oder ein (C₁-C₄)-Alkyl steht;
- R₃ für:
- ein (C₁-C₆)-Alkyl, das gegebenenfalls durch
a) ein oder mehrere Halogenatome;
b) einen nichtaromatischen (C₃-C₁₂)-Carbocyclus, der gesättigt oder ungesättigt und gegebenenfalls ein- oder mehrfach durch ein (C₁-C₄)-Alkyl substituiert ist;
c) ein Phenyl, das gegebenenfalls ein- oder mehrfach durch Substituenten, die unabhängig voneinander unter einem Halogenatom, einer Alk-Gruppe, einer OAlk-Gruppe, einer -S(O)ₙAlk-Gruppe, Nitro und Cyano ausgewählt sind, substituiert ist;
d) ein Naphthyl, das gegebenenfalls ein- oder mehrfach durch Substituenten, die unabhängig voneinander unter einem Halogenatom, einem (C₁-C₄)-Alkyl, einem (C₁-C₄)-Alkoxy und einem Trifluormethylrest ausgewählt sind, substituiert ist;
substituiert ist; oder
. Benzhydrylmethyl
steht;
- R₄ und R₅ jeweils unabhängig voneinander für ein Wasserstoffatom, ein Halogenatom, ein (C₁-C₄)-Alkyl, ein (C₁-C₄)-Alkomy, einen Trifluormethylrest oder Nitro stehen;
- R₇ für ein Wasserstoffatom oder ein (C₁-C₄)-Alkyl steht;
- R₈ für ein (C₁-C₄)-Alkyl oder einen Trifluormethylrest steht;
- R₉ und R₁₀ jeweils unabhängig voneinander für ein Wasserstoffatom oder ein (C₁-C₄)-Alkyl stehen;
- n für 0, 1 oder 2 steht;
- Alk für ein (C₁-C₄)-Alkyl, das gegebenenfalls ein- oder mehrfach durch ein Fluoratom substituiert ist, steht;
sowie ihre Salze oder ihre Solvate.

2. Verbindung der Formel (I) nach Anspruch 1, worin:
- X für ein Sauerstoffatom steht;
- R₁ für eine unter -NHSO₂CH₃ -NHSO₂CF₃, NHSO₂N(CH₃)₂, -S-CH₃, -SO-CH₃ und -SO₂-CH₃ ausgewählte Gruppe steht;
- R₂ für ein Wasserstoffatom, Methyl oder Ethyl steht;
- R₃ für:
. *tert*-Butyl, Isobutyl, 2,2-Dimethylpropyl, Adamantylmethyl, Phenylethyl, (2-Bromphenyl)-methyl, (2-Chlorphenyl)methyl, (2-Fluorphenyl)-methyl, (3-Methoxyphenyl)methyl, [2-(Trifluormethyl)phenyl]methyl, [3-(Trifluormethyl)phenyl]-methyl, [4-(Trifluormethyl)phenyl]methyl, (2,4-Dichlorphenyl) methyl, (2,3-Difluorphenyl)methyl, [2-Fluor-3-(trifluormethyl)phenyl]methyl, [2-Fluor-5-(trifluormethyl)phenyl]methyl, [3-Fluor-5-(trifluormethyl)phenyl]methyl, 1-Methyl-1-phenylethyl, 2-Phenylethyl, 2-(2-Chlorphenyl)ethyl, 3-Phenylpropyl, Naphthylmethyl, (2-Methylphenyl)-methyl, (2-Nitrophenyl)methyl, [3-(Trifluormethylthio)phenyl]methyl, (2-Methoxyphenyl)methyl, [2-(Trifluormethylthio)phenyl]methyl, [2-(Trifluormethoxy)phenyl]methyl oder (2-Cyanophenyl)methyl oder
. Benzhydrylmethyl
steht;
- R₄ für ein Wasserstoffatom steht oder sich in 6-Position des Indols befindet und für Chlor oder Methyl steht;
- R₅ für ein Wasserstoffatom steht oder sich in 7-position des Indols befindet und für ein Brom-, Chlor- oder Fluoratom, Methoxy oder Nitro steht; sowie ihre Salze oder ihre Solvate.

3. Verbindung der Formel (I) nach Anspruch 1, ausgewählt unter:
- N-[3-[7-Brom-3-(3,3-dimethylbutanoyl)-2-methyl-1*H*-indol-1-yl]propyl]methansulfonamid:
- N-[3-[7-Brom-2-methyl-3-[[2-(trifluormethyl)-phenyl]acetyl]-1*H*-indol-1-yl]propyl]methansulfonamid;
- N-[3-[7-Chlor-3-(3,3-dimethylbutanuyl)-2-methyl-1*H*-indol-1-yl]propyl]methansulfonamid:
- N-[3-[3-(1-Adamantylacetyl)-7-chlor-2-methyl-1*H-*indol-1-yl]propyl]methansulfonamid;
- N-[3-[3-[(2-Bromphenyl)acetyl]-7-chlor-2-methyl-1*H*-indol-1-yl]propyl]methansulfonamid;
- N-[3-[7-Chlor-3-[(2-chlorphenyl)acetyl]-2-methyl-1*H*-indol-1-yl]propyl]methansulfonamid;
- N-[3-[7-Chlor-2-methyl-3-[[2-(trifluormethyl)-phenyl]acetyl]-1*H*-indol-1-yl]propyl]methansulforamid;
- N-[3-[7-Chlor-3-[(2,3-difluorphenyl)acetyl]-2-methyl-1*H*-indol-1-yl]propyl]methansulfonamid;
- N-[3-[7-Fluor-2-methyl-3-[[2-(trifluormethyl)-phenyl]acetyl]-1*H*-indol-1-yl]propyl]methansulfonamid;
- N-[3-[7-Brom-3-(3,3-dimethylbutanoyl)-2,6-dimethyl-1*H*-indol-1-yl]propyl]methansulfonamid;
- N-[3-[7-Brom-3-[(2-chlorphenyl)acetyl]-2,6-dimethyl-1*H*-indol-1-yl]propyl]methansulfonamid;
- 1-[7-Chlor-2-methyl-1-[3-(methylthio)propyl]-1*H-*indol-3-yl)-2-[2-(trifluormethyl)phenyl]ethanon;
- N-[3-[7-Brom-3-[(2-chlorphenyl)acetyl]-2-methyl-1*H*-indol-1-yl]propyl]methansulfonamid;
- N-[3-[7-Brom-3-[(2-fluorphenyl)acetyl]-2-methyl-1*H*-indol-1-yl]propyl]methansulfonamid;
- N-[3-[7-Brom-2-methyl-3-[(2-methylphenyl)-acetyl]-1*H*-indol-1-yl]propyl]methansulfonamid:
- N-[3-[7-Brom-2-methyl-3-[(2-nitrophenyl)acetyl]-1*H*-indol-1-yl]propyl]methansulfonamid;
- N-[3-[7-Chlor-2-methyl-3-[(2-methylphenyl)-acetyl]-1H-indol-1-yl]propyl]methansulfonamid:
- N-[3-[7-Chlor-3-[(2-methoxyphenyl)acetyl]-2-methyl-1*H*-indol-1-yl]propyl]methansulfonamid;
- N-[3-[7-Chlor-2-methyl-3-[[2-[(trifluormethyl)-thio]phenyl]acetyl]-1*H*-indol-1-yl]propyl]methansulfonamid;
- N-[3-[3-[(2-Bromphenyl)acetyl]-7-fluor-2-methyl-1*H*-indol-1-yl]propyl]methansulfonamid;
- N-[3-[3-[(2-Chlorphenyl)acetyl]-7-fluor-2-methyl-1*H*-indol-1-yl]propyl]methansulfonamid;
- N-[-3-[7-Fluor-3-[(2-fluorphenyl)acetyl]-2-methyl-1*H*-indol-1-yl]propyl]methansulfonamid;
- N-[3-[7-Fluor-2-methyl-3-[(2-methylphenyl)-acetyl]-1*H*-indol-1-yl]propyl]methansulfonamid;
- N-[3-[7-Fluor-3-[(2-methoxyphenyl)acetyl]-2-methyl-1*H*-indol-1-yl]propyl]methansulfonamid;
- N-[3-[7-Brom-2,6-dimethyl-3-[[2-(trifluormethyl)phenyl]acetyl]-1*H*-indol-1-yl]propyl]methansulfonamid;
- N-[3-[3-[(2-Bromphenyl)acetyl]-7-methoxy-2-methyl-1*H*-indol-1-yl]propyl]methansulfonamid;
- N-[3-[7-Methoxy-2-methyl-3-[[2-(trifluormethyl)-phenyl]acetyl]-1*H*-indol-1-yl]propyl]methansulfonamid:
- N-[3-[3-[(2-Chlorphenyl)acetyl]-1-nitro-1*H-*indol-1-yl]propyl]methansulfonamid;
- N-[3-[3-[(2-Fluorphenyl)acetyl]-7-nitro-1*H-*indol-1-yl]propyl]methansulfonamid;
- N-[3-[3-[(2-Methylphenyl)acetyl]-7-nitro-1*H-*indol-1-yl]propyl]methansulfonamid;
- N-[3-[7-Nitro-3-[[2-(trifluormethyl)phenyl]-acetyl]-1*H*-indol-1-yl]propyl]methansulfonamid;
- N-[3-[3-[(2-Bromphenyl)acetyl]-7-nitro-1*H*-indol-1-yl]propyl]methansulfonamid;
- N-[3-[3-[(2-Methoxyphenyl)acetyl]-7-nitro-1*H-*indol-1-yl]propyl]methansulfonamid;
- N-[3-[7-Nitro-3-[[(2-(trifluormethoxy)phenyl)-acetyl]-1*H*-indol-1-yl]propyl]methansulfonamid;
- N-[3-[7-Nitro-3-[[(2-(nitrophenyl)acetyl]-1*H-*indol-1-yl]propyl]methansulfonamid:
- N-[3-[7-Brom-3-[(2-(bromphenyl)acetyl]-2-methyl-1*H*-indol-1-yl]propyl]methansulfonamid;
- N-[3-[7-Brom-3-[(2-(methoxyphenyl)acetyl]-2-methyl-1*H*-indol-1-yl]propyl]methansulfonamid;
- N-[3-[7-Brom-2-methyl-3-[[2-(trifluormethoxy)-phenyl]acetyl]-1*H*-indol-1-yl]propyl]methansulfonamid;
- N-[3-[7-Fluor-2-methyl-3-[[2-(trifluormethoxy)-phenyl]acetyl]-1*H*-indol-1-yl]propyl]methansulfonamid;
- N-[3-[7-Fluor-2-methyl-3-[(2-(nitrophenyl)-acetyl]-1*H*-indol-1-yl]propyl]methansulfonamid;
- 1,1,1-Trifluor-N-[3-[7-fluor-2-methyl-3-[(2-trifluormethyl)phenyl]acetyl]-1*H*-indol-1-yl]-propyl]methansulfonamid;
2-(1-Adamantyl)-1-[6,7-dichlor-2-methyl-1-[3-(methylthio)prvpyl]-1*H*-indol-3-yl]ethanon;
2-(1-Adamantyl)-1-[6,7-dichlor-2-methyl-1-[3-(methylsulfinyl)propyl]-1*H*-indol-3-yl]ethanon; sowie ihre Salze oder ihre Solvate.

4. Verfahren zur Herstellung der Verbindungen der Formel (I) nach Anspruch 1, worin X für ein Sauerstoffatom steht, **dadurch gekennzeichnet, dass** man: in Gegenwart einer Lewis-Säure eine Verbindung der Formel: 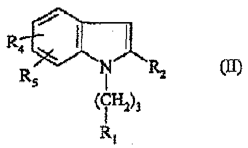 worin R₁, R₂, R₄ und R₅ wie für eine Verbindung der Formel (I) in Anspruch 1 definiert sind, mit einem Säurehalogenid der Formel: 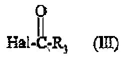 worin R₃ wie für eine Verbindung der Formel (I) in Anspruch 1 definiert ist und Hal für ein Halogenatom steht, umsetzt.

5. Medikament, **dadurch gekennzeichnet, dass** es eine Verbindung der Formel (I) nach einem der Ansprüche 1 bis 3 oder auch ein Hydrat oder ein Solvat der Verbindung der Formel (I) enthalt.

6. Pharmazeutische Zusammensetzung, **dadurch gekennzeichnet, dass** sie eine Verbindung der Formel (I) nach einem der Ansprüche 1 bis 3, ein Hydrat oder ein Solvat dieser Verbindung sowie mindestens einen pharmazeutisch akzeptablen Hilfsstoff umfasst.

7. Verwendung einer Verbindung der Formel (I) nach einem der Ansprüche 1 bis 3 zur Herstellung eines Medikaments zur Behandlung und Prävention von Autoimmunerkrankungen, allergischen Erkrankungen, Infektionserkrankungen, neurodegenerativen Erkrankungen, Herz-Kreislauf-Erkrankungen, Krebserkrankungen, Magen-Darm-Erkrankungen, Obesitas, Diabetes.
